# EUROPEAN PATENT SPECIFICATION

(11) **EP 3 231 365 B1**
(45) Date of publication and mention of the grant of the patent: **22.01.2025**
(21) Application number: 17172919.7
(22) Date of filing: 15.10.2009
(51) Int. Cl.: A61B 5/05

(54) **NEUROPHYSIOLOGIC MONITORING SYSTEM**
SYSTEM ZUR NEUROPHYSIOLOGISCHEN ÜBERWACHUNG
SYSTÈME DE SURVEILLANCE NEUROPHYSIOLOGIQUE

(30) Priority: 15.10.2008 US 196264 P
(43) Date of publication of application: 18.10.2017
(62) Divisional of application: 09820909.1
(73) Proprietor: Nuvasive, Inc., San Diego, CA 92121 (US)
(72) Inventor: GHARIB, James, San Diego, OR 92130 (US); FARQUHAR, Allen, deceased (US); LAYMAN, Doug, San Diego, CA 92129 (US); POTHIER, Albert, San Diego, CA 92106 (US)
(74) Representative: Morabito, Sara

(56) References cited:
- WO-A2-2006/084193
- WO-A2-2006/133015
- US-A1- 2007 021 682
- US-A1- 2008 071 191
- US-A1- 2008 167 574
- YONG HU ET AL: "Comparison of time-frequency analysis techniques in intraoperative somatosensory evoked potential (SEP) monitoring", COMPUTERS IN BIOLOGY AND MEDICINE, vol. 32, no. 1, 1 January 2002 (2002-01-01), pages 13 - 23, XP055094680, ISSN: 0010-4825, DOI: 10.1016/S0010-4825(01)00026-9

## Description

### FIELD

The present invention relates to a system generally aimed at surgery. More particularly, the present invention is directed at a system for performing surgical procedures and assessments involving the use of neurophysiologic recordings.

### BACKGROUND

Neurophysiology monitoring has become an increasingly important adjunct to surgical procedures where neural tissue may be at risk. Spinal surgery, in particular, involves working close to delicate tissue in and surrounding the spine, which can be damaged in any number of different ways. For example, an exiting nerve root may be comprised if surgical instruments have to pass near or close to the nerve while accessing the surgical target site in the spine. A spinal nerve and/or exiting nerve root may also be compromised if a pedicle screw, used often to secure fixation of multiple vertebra relative to each other, breaches the cortical layer of the pedicle. Surgeries targeting the spine may also require the retraction of nerve and/or vascular tissue out of the operative corridor. While doing so is necessary, there is a possibility of damaging nerve tissue through over retraction and/or a decreased supply of blood reaching the tissue due to the impingement of the retractor against the vascular tissue. Various neurophysiological techniques have been attempted and developed to monitor delicate nerve tissue during surgery in attempts to reduce the risk inherent in spine surgery (and surgery in general). Because of the complex structure of the spine and nervous system no single monitoring technique has been developed that may adequately assess the risk to nervous tissue in all situations and complex techniques are often utilized in conjunction one or more other complex monitoring techniques. EMG monitoring, for example, may be used to detect the presence of nerve roots near a surgical instrument or a breach formed in a pedicle wall. EMG monitoring is not, however, very effective when spinal cord monitoring is required.

When spinal cord monitoring is required, either or both motor evoked potential (MEP) or somatosensory evoked potential (SSEP) monitoring are often chosen. While both MEP and SSEP monitoring can be quite effective at detecting changes in the health of the spinal cord, MEP is limited because it only monitors the ventral column of the spinal cord and SSEP is limited because it only monitors the dorsal column of the spinal cord. Danger to nerve tissue that might then be detected using one these methods may be missed by the other, and vice versa. Thus, it may be most effective to use both MEP and SSEP monitoring during the same procedure, while still potentially needing EMG monitoring as well.

EMG, MEP, and SSEP involve complex analysis and specially trained neurophysiologists are generally called upon to perform the monitoring. Even though performed by specialists, interpreting the complex waveforms in this fashion is nonetheless disadvantageously prone to human error and can be disadvantageously time consuming, adding to the duration of the operation and translating into increased health care costs. Even more costly is the fact that the neurophysiologist is required in addition to the actual surgeon performing the spinal operation. Putting the difficulties associated with human interpretation of EMG, MEP, and SSEP monitoring aside, combining such testing in the OR generally requires multiple products to accommodate the differing requirements of each. This is disadvantageous when space is often at such a premium in the operating rooms of today. The present invention is directed at eliminating, or at least reducing the effects of, the above-described problems with the prior art.

WO 2006/084193 describes a system for SSEP monitoring of spinal cord health during surgery. The technical paper by Yong Ue et al in Computers in Biology and Medicine, volume 32, number 1, 1 January 2002, pages 13-23, XP005094680, describes time-frequency analyst techniques in SSEP monitoring. US 2008/167574 relates to an algorithm for finding stimulation thresholds in a neurophysiology monitoring system. WO2006/133015 describes a system for performing somatosensory evoked potential monitoring known in the art.

### SUMMARY OF THE INVENTION

The present invention provides a system for performing somatosensory evoked potential monitoring during surgery as set out in claim 1. Further advantageous aspects of the invention are set forth in the dependent claims

The present disclosure includes a system for avoiding harm to neural tissue during surgery. According to a broad aspect, the present invention includes instruments capable of stimulating either the peripheral nerves of a patient, the spinal cord of a patient, or both, additional instruments capable of recording the evoked somatosensory responses, and a processing system. The instrument is configured to deliver a stimulation signal preoperatively, perioperatively, and postoperatively. The processing system is programmed with a set of at least three threshold ranges and configured to receive first stimulation signal to said instrument at a first magnitude. The first magnitude corresponds to a boundary between the pair of ranges. The processing system further receives a second stimulation signal at a second magnitude corresponding to a boundary between a different pair of the ranges. The processing unit is still further programmed to and measure the response of nerves depolarized by said stimulation signals as received by the somatosensory cortex to indicate spinal cord health.

According to another broad aspect, the present invention includes a control unit, a patient module, and a plurality of surgical accessories adapted to couple to the patient module. The control unit includes a power supply and is programmed to receive user commands, activate stimulation in a plurality of predetermined modes, process signal data according to defined algorithms, display received parameters and processed data, and monitor system status. The patient module is in communication with the control unit. The patient module is within the sterile field. The patient module includes signal conditioning circuitry, stimulator drive circuitry, and signal conditioning circuitry required to perform said stimulation in said predetermined modes. The patient module includes a processor programmed to perform a plurality of predetermined functions using said instrument including at least two of static pedicle integrity testing, dynamic pedicle integrity testing, nerve proximity detection, neuromuscular pathway assessment, manual motor evoked potential monitoring, automatic motor evoked potential monitoring, manual somatosensory evoked potential monitoring, automatic somatosensory evoked potential monitoring, non-evoked monitoring, and surgical navigation.

According to still another broad aspect, the present invention includes an instrument and a processing system. The instrument is in communication with the processing unit. The instrument is capable of advancement to a surgical target site and is configured to deliver a stimulation signal at least one of while advancing to said target site and after reaching said target site. The processing unit is programmed to perform a plurality of predetermined functions using said instrument including at least two of static pedicle integrity testing, dynamic pedicle integrity testing, nerve proximity detection, neuromuscular pathway assessment, manual motor evoked potential monitoring, automatic motor evoked potential monitoring, manual somatosensory evoked potential monitoring, automatic somatosensory evoked potential monitoring, non-evoked monitoring, and surgical navigation. The processing system has a preestablished profile for at least one of said predetermined functions so as to facilitate the initiation of said at least one predetermined function.

### BRIEF DESCRIPTION OF THE DRAWINGS

Many advantages of the present invention will be apparent to those skilled in the art with a reading of this specification in conjunction with the attached drawings, wherein like reference numerals are applied to like elements and wherein:
Figure 1 is a block diagram of an exemplary surgical system capable of conducting multiple nerve and spinal cord monitoring functions including but not necessarily limited to SSEP Manual, SSEP Automatic, MEP Manual, MEP Automatic, neuromuscular pathway, bone integrity, nerve detection, and nerve pathology (evoked or free-run EMG) assessments;
Fig. 2 is a perspective view showing examples of several components of the neurophysiology system of Fig. 1;
Fig. 3 is a perspective view of an example of a control unit forming part of the neurophysiology system of Fig. 1;
Figs. 4-6 are perspective, top, and side views, respectively, of an example of a patient module forming part of the neurophysiology system of Fig. 1;
Fig. 7 is a top view of an electrode harness forming part of the neurophysiology system of Fig. 1;
Figs. 8A-8C are side views of various examples of harness ports forming part of the neurophysiology system of Fig. 1;
Fig. 9 is a plan view of an example of a label affixed to an electrode connector forming part of the neurophysiology system of Fig. 1;
Figs 10A-10B are top views of examples of electrode caps forming part of the neurophysiology system of Fig. 1;
Figs. 11-12 are perspective views of an example of a secondary display forming part of the neurophysiology system of Fig. 1;
Fig. 13 is an exemplary screen display illustrating one embodiment of a general system setup screen forming part of the neurophysiology system of Fig. 1;
Fig. 14 is an exemplary screen display illustrating one embodiment of a detailed profile screen forming part of the neurophysiology system of Fig. 1;

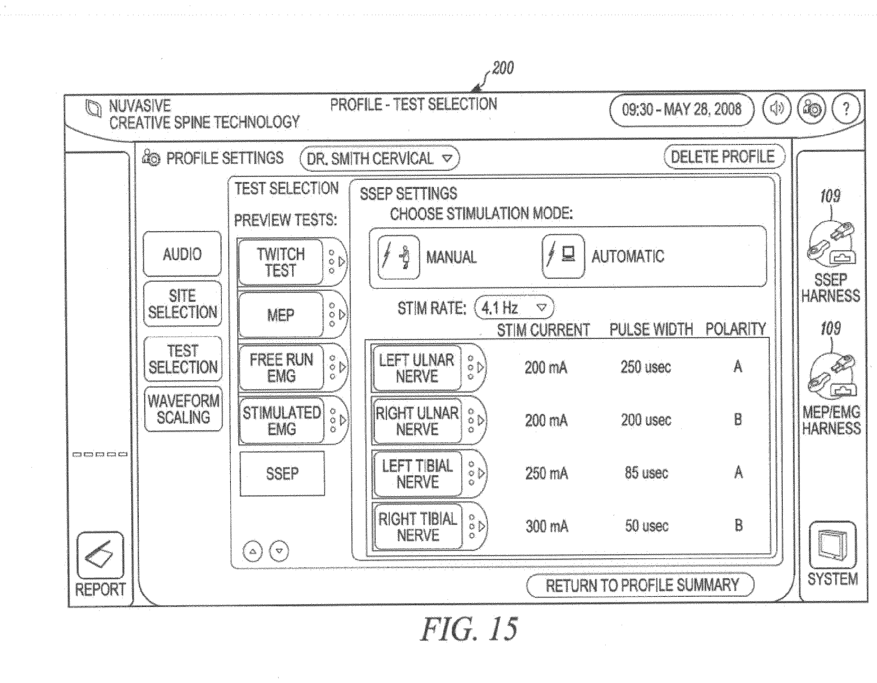

Fig. 15 is an exemplary screen display illustrating one embodiment of a custom profile selection screen forming part of the neurophysiology system of Fig. 1;
Fig. 16 is an exemplary screen display with features of an electrode test as implemented in one embodiment of an electrode test screen forming part of the neurophysiology system of Fig. 1;
Fig. 17 is an exemplary screen display illustrating one embodiment of an SSEP profile selection screen forming part of the neurophysiology system of Fig. 1;
Fig. 18 is an exemplary screen display illustrating a second embodiment of a SSEP Manual Stimulus Mode setting with a Left Ulnar Nerve (LUN) Breakout screen forming part of the neurophysiology system of Fig. 1;
Fig. 19 is an exemplary screen display illustrating one embodiment of an SSEP Manual Run screen forming part of the neurophysiology system of Fig. 1;
Fig. 20 is an exemplary screen display illustrating a second embodiment of an SSEP Manual Run screen forming part of the neurophysiology system of Fig. 1;
Fig. 21 is an exemplary screen display illustrating a third embodiment of an SSEP Manual Run screen forming part of the neurophysiology system of Fig. 1;
Fig. 22 is an exemplary screen display illustrating a fourth embodiment of an SSEP Manual Run screen forming part of the neurophysiology system of Fig. 1;
Fig. 23 is an exemplary screen display illustrating one embodiment of an SSEP Automatic Test Setting screen forming part of the neurophysiology system of Fig. 1;
Fig. 24 is an exemplary screen display illustrating one embodiment of an SSEP Automatic Run screen forming part of the neurophysiology system of Fig. 1;
Fig. 25 is an exemplary screen display illustrating a second embodiment of an SSEP Automatic Run screen forming part of the neurophysiology system of Fig. 1;
Fig. 26 is an exemplary screen display illustrating a third embodiment of an SSEP Automatic Run screen forming part of the neurophysiology system of Fig. 1;
Fig. 27 is a screen shot of an example of a Manual MEP monitoring screen forming part of the neurophysiology system of Fig. 1;
Fig. 28 is a screen shot of an example of an Automatic MEP monitoring screen forming part of the neurophysiology system of Fig. 1;
Fig. 29 is a screenshot of an example of a Twitch Test monitoring screen forming part of the neurophysiology system of Fig. 1;
Fig. 30 is a screenshot of an example of a Basic Stimulation EMG monitoring screen forming part of the neurophysiology system of Fig. 1;
Fig. 31 is a screenshot of an example of a dynamic stimulation EMG monitoring screen forming part of the neurophysiology system of Fig. 1;
Fig. 32 is a screenshot of an example of a Nerve Surveillance EMG monitoring screen forming part of the neurophysiology system of Fig. 1;
Fig. 33 is a screenshot of an example of a Free-Run EMG monitoring screen forming part of the neurophysiology system of Fig. 1;
Fig. 34 is a screenshot of an example of a Navigated Guidance screen forming part of the neurophysiology system of Fig. 1;
Figs. 35 A-D are graphs illustrating the fundamental steps of a rapid current threshold-hunting algorithm according to one embodiment of the present invention;
Fig. 36 is block diagram illustrating the steps of an initiation sequence for determining a relevant safety level prior to determining a precise threshold value according to an alternate embodiment of the threshold hunting algorithm of Fig. 35 A-D;
Fig. 37 is a flowchart illustrating the method by which a multi-channel hunting algorithm determines whether to perform or omit a stimulation;
Fig. 38 A-C are graphs illustrating use of the threshold hunting algorithm of Fig.39 and further omitting stimulations when the likely result is already clear from previous data;
Fig. 39 A is a flowchart illustrating the sequence employed by the algorithm to determine and monitor Ithresh;
Fig. 39 B is a graph illustrating the confirmation step employed by the algorithm to determine whether Ithresh has changed from a previous determination;
Fig. 40 is a flow chart indicating the steps used to automatically determine optimized parameters for SSEP peripheral nerve stimulation for all four limbs; and
Fig. 41 is a flow chart indicating the steps used to automatically determine optimized parameters for SSEP peripheral nerve stimulation for one limb utilizing a threshold determination algorithm.

### DETAILED DESCRIPTION OF THE PREFERRED EMBODIMENT

Illustrative embodiments of the invention are described below. In the interest of clarity, not all features of an actual implementation are described in this specification. It will of course be appreciated that in the development of any such actual embodiment, numerous implementation-specific decisions must be made to achieve the developers' specific goals, such as compliance with system-related and business-related constraints, which will vary from one implementation to another. Moreover, it will be appreciated that such a development effort might be complex and time-consuming, but would nevertheless be a routine undertaking for those of ordinary skill in the art having the benefit of this disclosure. The systems disclosed herein boast a variety of inventive features and components that warrant patent protection, both individually and in combination. It is also expressly noted that, although described herein largely in terms of use in spinal surgery, the surgical system and related methods described herein are suitable for use in any number of additional procedures, surgical or otherwise, wherein assessing the health of the spinal cord and/or various other nerve tissue may prove beneficial.

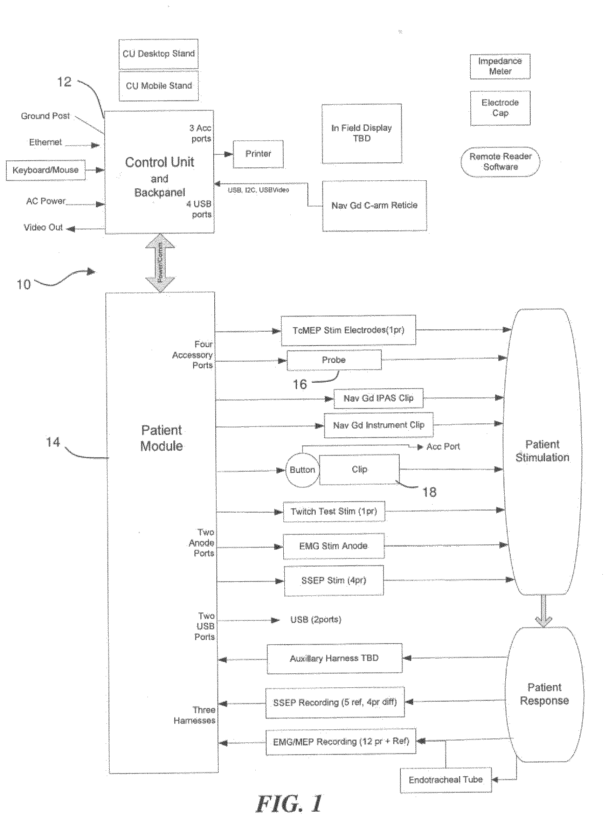

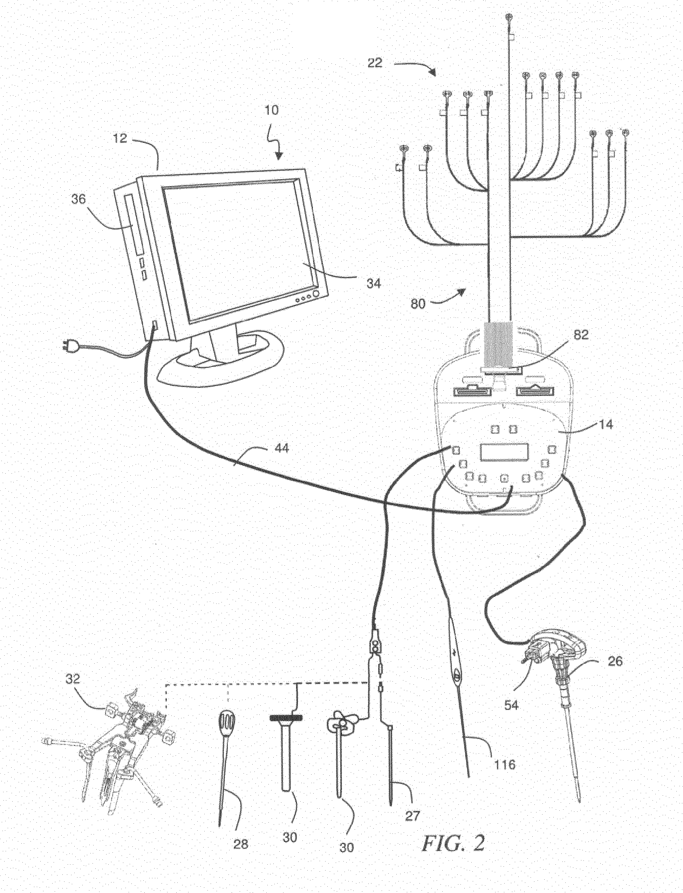

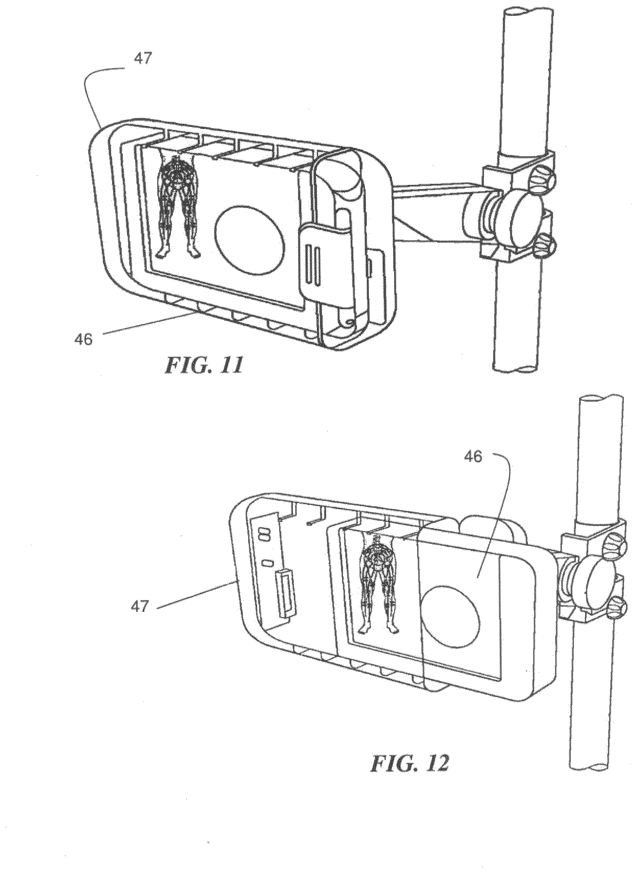

A surgeon operable neurophysiology system 10 is described herein and is capable of performing a number of neurophysiological and/or guidance assessments at the direction of the surgeon (and/or other members of the surgical team). By way of example only, Figs. 1-2 illustrate the basic components of the neurophysiology system 10. The system comprises a control unit 12 (including a main display 34 preferably equipped with a graphical user interface (GUI) and a processing unit 36 that collectively contain the essential processing capabilities for controlling the system 10), a patient module 14, a stimulation accessory (e.g. a stimulation probe 16, stimulation clip 18 for connection to various surgical instruments, an inline stimulation hub 20, and stimulation electrodes 22), and a plurality of recording electrodes 24 for detecting electrical potentials. The stimulation clip 18 may be used to connect any of a variety of surgical instruments to the system 10, including, but not necessarily limited to a pedicle access needle 26, k-wire 27, tap 28, dilator(s) 30, tissue retractor 32, etc. One or more secondary feedback devices (e.g. secondary display 46 in Fig. 11-12) may also be provided for additional expression of output to a user and/or receiving input from the user.

In one embodiment, the neurophysiology system 10 may be configured to execute any of the functional modes including, but not necessarily limited to, static pedicle integrity testing ("Basic Stimulated EMG"), dynamic pedicle integrity testing ("Dynamic Stimulated EMG"), nerve proximity detection ("XLIF^{®}"), neuromuscular pathway assessment ("Twitch Test"), motor evoked potential monitoring ("MEP Manual" and "MEP Automatic"), somatosensory evoked potential monitoring ("SSEP Manual" and "SSEP Automatic"), non-evoked monitoring ("Free-run EMG") and surgical navigation ("Navigated Guidance"). The neurophysiology system 10 may also be configured for performance in any of the lumbar, thoracolumbar, and cervical regions of the spine.

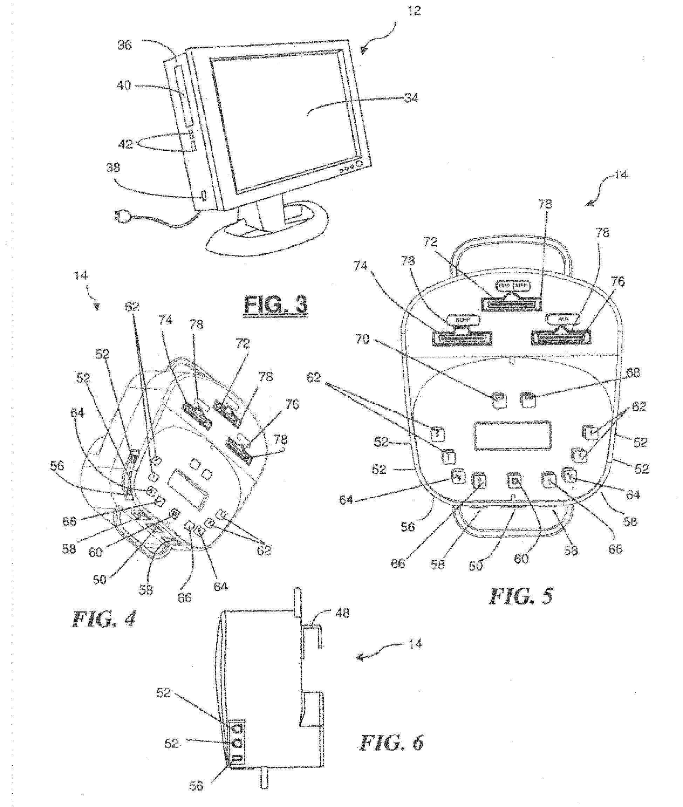

Before further addressing the various functional modes of the surgical system 10, the hardware components and features of the system 10 will be describe in further detail. The control unit 12 of the neurophysiology system 10, illustrated by way of example only in Fig. 3, includes a main display 34 and a processing unit 36, which collectively contain the essential processing capabilities for controlling the neurophysiology system 10. The main display 34 is preferably equipped with a graphical user interface (GUI) capable of graphically communicating information to the user and receiving instructions from the user. The processing unit 36 contains computer hardware and software that commands the stimulation source (e.g. patient module 14, Figs. 4-6), receives digital and/or analog signals and other information from the patient module 14, processes EMG and SSEP response signals, and displays the processed data to the user via the display 34. The primary functions of the software within the control unit 12 include receiving user commands via the touch screen main display 34, activating stimulation in the appropriate mode (Basic Stimulated EMG, Dynamic Stimulated EMG, XLIF, MEP automatic, MEP manual, SSEP manual, SSEP auto, and Twitch Test), processing signal data according to defined algorithms, displaying received parameters and processed data, and monitoring system status. According to one example embodiment, the main display 34 may comprise a 15" LCD display equipped with suitable touch screen technology and the processing unit 36 may comprise a 2GHz. The processing unit 36 shown in Fig. 3 further includes a powered USB port 38 for connection to the patient module 14, a media drive 40 (e.g. CD, CD-RW, DVD, DVD-RW, etc...), a network port, wireless network card, and a plurality of additional ports 42 (e.g. USB, IEEE 1394, infrared, etc...) for attaching additional accessories, such as for example only, navigated guidance sensors, auxiliary stimulation anodes, and external devices (e.g. printer, keyboard, mouse, etc...). Preferably, during use the control unit 12 sits near the surgical table but outside the surgical field, such as for example, on a table top or a mobile stand. It will be appreciated, however, that if properly draped and protected, the control unit 12 may be located within the surgical (sterile) field.

The patient module 14, shown by way of example only in FIGS. 4-6, is communicatively linked to the control unit 12. In this embodiment the patient module 14 is communicatively linked with and receives power from the control unit 12 via a USB data cable 44. However, it will be appreciated that the patient module 14 may be supplied with its own power source and other known data cables, as well as wireless technology, may be utilized to establish communication between the patient module 14 and control unit 12. The patient module 14 contains a digital communications interface to communicate with the control unit 12, as well as the electrical connections to all recording and stimulation electrodes, signal conditioning circuitry, stimulator drive and steering circuitry, and signal conditioning circuitry required to perform all of the functional modes of the neurophysiology system 10, including but not necessarily limited to Basic Stimulated EMG, Dynamic Stimulated EMG, XLIF^{®}, Twitch Test, MEP Manual and MEP Automatic, and SSEP. In one example, the patient module 14 includes thirty-two recording channels and eleven stimulation channels. A display (e.g. an LCD screen) may be provided on the face of the patient module 14, and may be utilized for showing simple status readouts (for example, results of a power on test, the electrode harnesses attached, and impedance data, etc...) or more procedure related data (for example, a stimulation threshold result, current stimulation level, selected function, etc ...). The patient module 14 may be positioned near the patient in the sterile field during surgery. By way of example, the patient module 14 may be attached to bed rail with the aid of a hook 48 attached to, or forming a part of, the patient module 14 casing.

With reference to Figs. 4-6, patient module 14 comprises a multitude of ports and indicators for connecting and verifying connections between the patient module 14 and other system components. A control unit port 50 is provided for data and power communication with the control unit 12, via USB data cable 44 as previously described. There are four accessory ports 52 provided for connecting up to the same number of surgical accessories, including, but not necessarily limited to, stimulation probe 16, stimulation clip 18, inline stimulation hub 20, and navigated guidance sensor (or tilt sensor) 54. The accessory ports 52 include a stimulation cathode and transmit digital communication signals, tri-color LED drive signals, button status signals, identification signals, and power between the patient module 14 and the attached accessory. A pair of anode ports 56, preferably comprising 2 wire DIN connectors, may be used to attach auxiliary stimulation anodes should it become desirable or necessary to do so during a procedure. A pair of USB ports 58 are connected as a USB hub to the control unit 12 and may be used to make any number of connections, such as for example only, a portable storage drive.

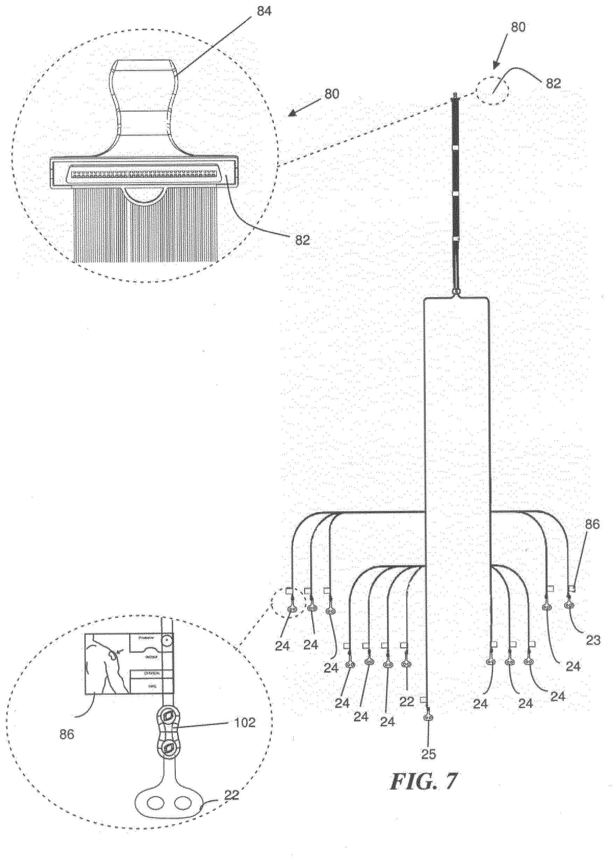

As soon as a device is plugged into any one of ports 50, 52, 56, or 58, the neurophysiology system 10 automatically performs a circuit continuity check to ensure the associated device will work properly. Each device forms a separate closed circuit with the patient module such that the devices may be checked independent of each other. If one device is not working properly the device may be identified individually while the remaining devices continue indicate their valid status. An indicator LED is provided for each port to convey the results of the continuity check to the user. Thus, according to the example embodiment of Figs. 7-9, the patient module 14 includes one control unit indicator 60, four accessory indicators 62, two anode indicators 64, and two USB indicators 66. According to a preferred embodiment, if the system detects an incomplete circuit during the continuity check, the appropriate indicator will turn red alerting the user that the device might not work properly. On the other hand, if a complete circuit is detected, the indicator will appear green signifying that the device should work as desired. Additional indicator LEDs are provided to indicate the status of the system and the MEP stimulation. The system indicator 68 will appear green when the system is ready and red when the system is not ready. The MEP stim indicator 70 lights up when the patient module is ready to deliver and MEP stimulation signal. In one embodiment, the MEP stim indicator 68 appears yellow to indicate a ready status.

To connect the array of recording electrodes 24 and stimulation electrodes 22 utilized by the system 10, the patient module 14 also includes a plurality of electrode harness ports. In the embodiment shown, the patient module 14 includes an EMG/MEP harness port 72, SSEP harness port 74, and an Auxiliary harness port 76 (for expansion and/or custom harnesses). Each harness port 72, 74, and 76 includes a shaped socket 78 that corresponds to a matching shaped connector 82 on the appropriate electrode harness 80. In addition, the neurophysiology system 10 may preferably employ a color code system wherein each modality (e.g. EMG, EMG/MEP, and SSEP) has a unique color associated with it. By way of example only and as shown herein, EMG monitoring (including, screw tests, detection, and nerve retractor) may be associated with the color green, MEP monitoring with the color blue, and SSEP monitoring may be associated with the color orange. Thus, each harness port 72, 74, 76 is marked with the appropriate color which will also correspond to the appropriate harness 80. Utilizing the combination of the dedicated color code and the shaped socket/connector interface simplifies the setup of the system, reduces errors, and can greatly minimize the amount of pre-operative preparation necessary. The patient module 14, and especially the configuration of quantity and layout of the various ports and indicators, has been described according to one example embodiment of the present invention. It should be appreciated, however, that the patient module 14 could be configured with any number of different arrangements without departing from the scope of the invention as claimed.

As mentioned above, to simplify setup of the system 10, all of the recording electrodes 24 and stimulation electrodes 22 that are required to perform one of the various functional modes (including a common electrode 23 providing a ground reference to pre-amplifiers in the patient module 14, and an anode electrode 25 providing a return path for the stimulation current) are bundled together and provided in single electrode harness 80, as illustrated, by way of example only, in Fig. 7. Depending on the desired function or functions to be used during a particular procedure, different groupings of recoding electrodes 24 and stimulation electrodes 22 may be required. By way of example, the SSEP function requires more stimulating electrodes 22 than either the EMG or MEP functions, but also requires fewer recording electrodes than either of the EMG and MEP functions. To account for the differing electrode needs of the various functional modes, the neurophysiology system 10 may employ different harnesses 80 tailored for the desired modes. According to one embodiment, three different electrode harnesses 80 may be provided for use with the system 10, an EMG harness, an EMG/MEP harness, and an SSEP harness.

At one end of the harness 80 is the shaped connector 82. As described above, the shaped connector 82 interfaces with the shaped socket 72, 74, or 76 (depending on the functions harness 80 is provided for). Each harness 80 utilizes a shaped connector 82 that corresponds to the appropriate shaped socket 72, 74, 76 on the patient module 14. If the shapes of the socket and connector do not match the harness 80, connection to the patient module 14 cannot be established. According to one embodiment, the EMG and the EMG/MEP harnesses both plug into the EMG/MEP harness port 72 and thus they both utilize the same shaped connector 82.

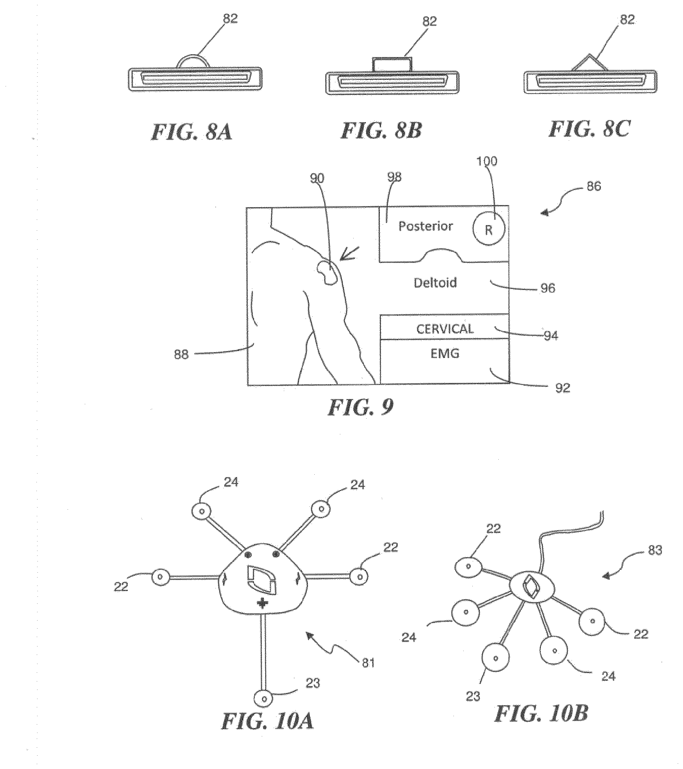

By way of example only, Figs. 8A-8C illustrate the various shape profiles used by the different harness ports 72, 74, 76 and connectors 82. Fig. 8A illustrates the half circular shape associated with the EMG and EMG/MEP harness and port 72. Fig. 8B illustrates the rectangular shape utilized by the SSEP harness and port 74. Finally, Fig. 8C illustrates the triangular shape utilized by the Auxiliary harness and port 76. Each harness connector 82 includes a digital identification signal that identifies the type of harness 80 to the patient module 14. At the opposite end of the electrode harness 80 are a plurality of electrode connectors 102 linked to the harness connector 82 via a wire lead. Using the electrode connector 102, any of a variety of known electrodes may be used, such as by way of example only, surface dry gel electrodes, surface wet gel electrodes, and needle electrodes.

To facilitate easy placement of scalp electrodes used during MEP and SSEP modes, an electrode cap 81, depicted by way of example only in Fig. 10A may be used. The electrode cap 81 includes two recording electrodes 23 for SSEP monitoring, two stimulation electrodes 22 for MEP stimulation delivery, and an anode 23. Graphic indicators may be used on the electrode cap 81 to delineate the different electrodes. By way of example, lightning bolts may be used to indicate a stimulation electrode, a circle within a circle may be used to indicate recording electrodes, and a stepped arrow may be used to indicate the anode electrode. The anode electrode wire is colored white to further distinguish it from the other electrodes and is significantly longer that the other electrode wires to allow placement of the anode electrode on the patient's shoulder. The shape of the electrode cap 81 may also be designed to simplify placement. By way of example only, the cap 81 has a pointed end that may point directly toward the patient's nose when the cap 81 is centered on the head in the right orientation. A single wire may connect the electrode cap 81 to the patient module 14 or electrode harness 80, thereby decreasing the wire population around the upper regions of the patient. Alternatively, the cap 81 may be equipped with a power supply and a wireless antenna for communicating with the system 10. Fig. 10B illustrates another example embodiment of an electrode cap 83 similar to cap 81. Rather than using graphic indicators to differentiate the electrodes, colored wires may be employed. By way of example, the stimulation electrodes 22 are colored yellow, the recording electrodes 24 are gray, and the anode electrode 23 is white. The anode electrode is seen here configured for placement on the patient's forehead. According to an alternate embodiment, the electrode cap (not shown) may comprise a strap or set of straps configured to be worn on the head of the patient. The appropriate scalp recording and stimulation sites may be indicated on the straps. By way of example, the electrode cap may be imbued with holes overlying each of the scalp recording sites (for SSEP) and scalp stimulation sites (for MEP). According to a further example embodiment, the border around each hole may be color coded to match the color of an electrode lead wire designated for that site. In this instance, the recording and stimulation electrodes designated for the scalp are preferably one of a needle electrode and a corkscrew electrode that can be placed in the scalp through the holes in the cap.

In addition to or instead of color coding the electrode lead wires to designated intended placement, the end of each wire lead next to the electrode connector 102 may be tagged with a label 86 that shows or describes the proper positioning of the electrode on the patient. The label 86 preferably demonstrates proper electrode placement graphically and textually. As shown in Fig. 9, the label may include, a graphic image showing the relevant body portion 88 and the precise electrode position 90. Textually, the label 86 may indicate the side 100 and muscle (or anatomic location) 96 for placement, the function of the electrode (e.g. stimulation, recording channel, anode, and reference - not shown), the patient surface (e.g. anterior or posterior), the spinal region 94, and the type of monitoring 92 (e.g. EMG, MEP, SSEP, by way of example, only). According to one embodiment (set forth by way of example only), the electrode harnesses 80 are designed such that the various electrodes may be positioned about the patient (and preferably labeled accordingly) as described in Table 1 for Lumbar EMG, Table 2 for Cervical EMG, Table 3 for Lumbar/Thoracolumbar EMG and MEP, Table 4 for Cervical EMG and MEP, and Table 5 for SSEP:

**Table 1: Lumbar EMG**

| **Electrode Type** | **Electrode Placement** | **Spinal Level** |
|---|---|---|
| Ground | Upper Outer Thigh | - |
| Anode | Latissimus Dorsi | - |
| Stimulation | Knee | - |
| Recording | Left Tibialis Anterior | L4, L5 |
| Recording | Left Gastroc. Medialis | S1, S2 |
| Recording | Left Vastus Medialis | L2, L3, L4 |
| Recording | Left Biceps Femoris | L5, S1, S2 |
| Recording | Right Biceps Femoris | L5, S1, S2 |
| Recording | Right Vastus Medialis | L2, L3, L4 |
| Recording | Right Gastroc. Medialis | S1, S2 |
| Recording | Right Tibialis Anterior | L4, L5 |

**Table 2: Cervical EMG**

| **Electrode Type** | **Electrode Placement** | **Spinal Level** |
|---|---|---|
| Ground | Shoulder | - |
| Anode | Mastoid | - |
| Stimulation | Inside Elbow | - |
| Recording | Left Triceps | C7, C8 |
| Recording | Left Flexor Carpi Radialis | C6, C7, C8 |
| Recording | Left Deltoid | C5, C6 |
| Recording | Left Trapezius | C3, C4 |
| Recording | Left Vocal Cord | RLN |
| Recording | Right Vocal Cord | RLN |
| Recording | Right Trapezius | C3, C4 |
| Recording | Right Deltoid | C5, C6 |
| Recording | Right Flexor Carpi Radialis | C6, C7, C8 |
| Recording | Right Triceps | C7, C8 |

**Table 3: Lumbar/Thoracolumbar EMG + MEP**

| **Electrode Type** | **Electrode Placement** | **Spinal Level** |
|---|---|---|
| Ground | Upper Outer Thigh | - |
| Anode | Latissimus Dorsi | - |
| Stimulation | Knee | - |
| Recording | Left Tibialis Anterior | L4, L5 |
| Recording | Left Gastroc. Medialis | S1, S2 |
| Recording | Left Vastus Medialis | L2, L3, L4 |
| Recording | Left Biceps Femoris | L5, S1, S2 |
| Recording | Left APB-ADM | C6, C7, C8, T1 |
| Recording | Right APB-ADM | C6, C7, C8, T1 |
| Recording | Right Biceps Femoris | L5, S1, S2 |
| Recording | Right Vastus Medialis | L2, L3, L4 |
| Recording | Right Gastroc. Medialis | S1, S2 |
| Recording | Right Tibialis Anterior | L4, L5 |

**Table 4: Cervical EMG + MEP**

| **Electrode Type** | **Electrode Placement** | **Spinal Level** |
|---|---|---|
| Ground | Shoulder | - |
| Anode | Mastoid | - |
| Stimulation | Inside Elbow | - |
| Recording | Left Tibialis Anterior | L4, L5 |
| Recording | Left Flexor Carpi Radialis | C6, C7, C8 |
| Recording | Left Deltoid | C5, C6 |
| Recording | Left Trapezius | C3, C4 |
| Recording | Left APB-ADM | C6, C7, C8, T1 |
| Recording | Left Vocal Cord | RLN |
| Recording | Right Vocal Cord | RLN |
| Recording | Right APB-ADM | C6, C7, C8, T1 |
| Recording | Right Trapezius | C3, C4 |
| Recording | Right Deltoid | C5, C6 |
| Recording | Right Flexor Carpi Radialis | C6, C7, C8 |
| Recording | Right Tibialis Anterior | L4, L5 |

**Table 5: SSEP**

| **Electrode Type** | **Electrode Placement** | **Spinal Level** |
|---|---|---|
| Ground | Shoulder | - |
| Stimulation | Left Post Tibial Nerve | - |
| Stimulation | Left Ulnar Nerve | - |
| Stimulation | Right Post Tibial Nerve | - |
| Stimulation | Right Ulnar Nerve | - |
| Recording | Left Popliteal Fossa | - |
| Recording | Left Erb's Point | - |
| Recording | Left Scalp Cp3 | - |
| Recording | Right Popliteal Fossa | - |
| Recording | Right Erb's Point | - |
| Recording | Right Scalp Cp4 | - |
| Recording | Center Scalp Fpz | - |
| Recording | Center Scalp Cz | - |
| Recording | Center Cervical Spine | - |

As mentioned above, the neurophysiology monitoring system 10 may include a secondary display, such as for example only, the secondary display 46 illustrated in Figs. 11-12. The secondary display 46 may be configured to display some or all of the information provided on main display 34. The information displayed to the user on the secondary display 34 may include, but is not necessarily limited to, alpha-numeric and/or graphical information regarding any of the selected function modes (e.g. SSEP Manual, SSEP Automatic, MEP Manual, MEP Automatic, Twitch Test, Basic Stimulated EMG, Dynamic Stimulated EMG, XLIF, Free-Run EMG, and Navigated Guidance), attached accessories (e.g. stimulation probe 16, stimulation clip 18, tilt sensor 54) , electrode harness or harnesses attached, impedance test results, myotome/EMG levels, stimulation levels, history reports, selected parameters, test results, etc... In one embodiment, secondary display 46 may be configured to receive user input in addition to its display function. The secondary display 46 can thus be used as an alternate control point for the system 10. The control unit 12 and secondary display 46 may be linked such that input may be received on from one display without changing the output shown on the other display. This would allow the surgeon to maintain focus on the patient and test results while still allowing other members of the OR staff to manipulate the system 10 for various purposes (e.g. inputting annotations, viewing history, etc...). The secondary display 46 may be battery powered. Advantageously, the secondary display 46 may be positioned inside the sterile field as well as outside the sterile field. For positioning within the sterile field a disposable sterile case 47 may be provided to house the display. Alternatively, the display 46 may be sterile bagged. Both the sterile case 47 and the secondary display 46 may be mounted to a pole, bed frame, light fixture, or other apparatus found near and/or in the surgical field. It is further contemplated that multiple secondary displays 46 may be linked to the control unit 12. This may effectively distribute neurophysiology information and control throughout the operating room. By way of example, a secondary display 46 may also be provided for the anesthesiologist. This may be particularly useful in providing the anesthesiologist with results from the Twitch Test and providing reminders about the use of paralytics, which may adversely affect the accuracy of the neurophysiology system 10. Wired or wireless technology may be utilized to link the secondary display 46 to the control unit 12.

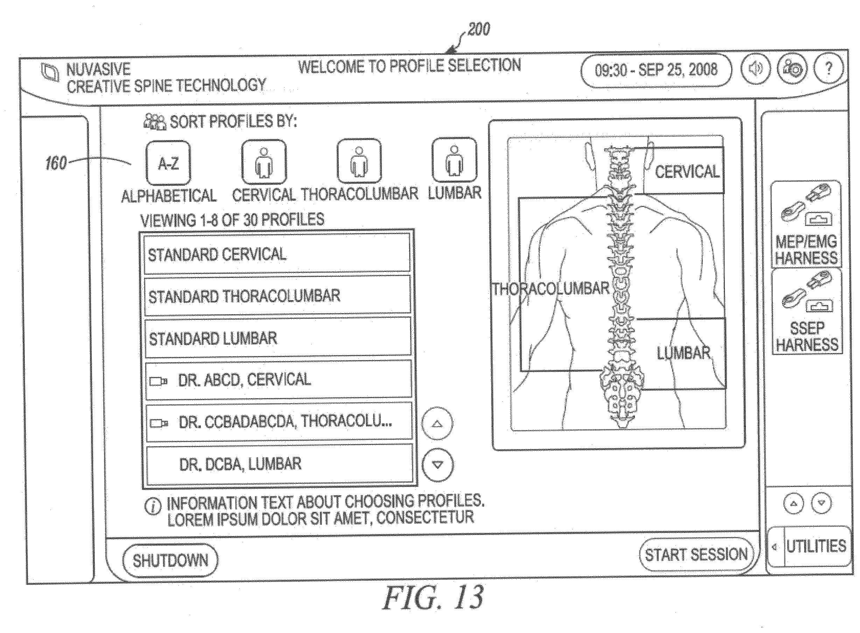

Having described an example embodiment of the system 10 and the hardware components that comprise it, the neurophysiological functionality and methodology of the system 10 will now be described in further detail. Various parameters and configurations of the neuromonitoring system 10 may depend upon the target location (i.e. spinal region) of the surgical procedure and/or user preference. In one embodiment, upon starting the system 10 the software will open to a startup screen, illustrated by way of example only, in Fig. 13. The startup screen includes a profile selection window 160 from which the user may select from one of the standard profiles (e.g. "Standard Cervical," "Standard Thoracolumbar," and "Standard Lumbar") or any custom profiles that have been previously saved to the system. Profiles may be arranged for selection, alphabetically, by spinal region, or by other suitable criteria. Profiles may be saved to the control unit hard drive or to a portable memory device, such as for example, a USB memory drive, or on a web server.

Selecting a profile configures the system 10 to the parameters assigned for the selected profile (standard or custom). The availability of different function modes may depend upon the profile selected. By way of example only, selecting the cervical and thoracolumbar spinal regions may automatically configure the options to allow selection of the SSEP Manual, SSEP Automatic, MEP Manual, MEP Automatic, Twitch Test, Basic Stimulated EMG, Dynamic Stimulated EMG, XLIF, Free-Run EMG, and Navigated Guidance modes, while selecting the lumbar region may automatically configure the options to allow selection of the Twitch Test, Basic, Difference, and Dynamic Stimulated EMG Tests, XLIF^{®}, and Nerve Retractor modes. Default parameters associated with the various function modes may also depend on the profile selected, for example, the characteristics of the stimulation signal delivered by the system 10 may vary depending on the profile. By way of example, the stimulation signal utilized for the Stimulated EMG modes may be configured differently when a lumbar profile is selected versus when one of a thoracolumbar profile and a cervical profile.

As previously described above, each of the hardware components includes an identification tag that allows the control unit 12 to determine which devices are hooked up and ready for operation. In one embodiment, profiles may only be available for selection if the appropriate devices (e.g. proper electrode harness 80 and stimulation accessories) are connected and/or ready for operation. Alternatively, the software could bypass the startup screen and jump straight to one of the functional modes based on the accessories and/or harnesses it knows are plugged in. The ability to select a profile based on standard parameters, and especially on customized preferences, may save significant time at the beginning of a procedure and provides for monitoring availability right from the start. Moving on from the startup screen, the software advances directly to an electrode test screen and impedance tests, which are performed on every electrode as discussed above. When an acceptable impedance test has been completed, the system 10 is ready to begin monitoring and the software advances to a monitoring screen from which the neurophysiological monitoring functions of the system 10 are performed.

The information displayed on the monitoring screen may include, but is not necessarily limited to, alpha-numeric and/or graphical information regarding any of the functional modes (e.g. SSEP Manual, SSEP Automatic, MEP Manual, MEP Automatic, Twitch Test, Basic Stimulated EMG, Dynamic Stimulated EMG, XLIF, Free-Run EMG, and Navigated Guidance), attached accessories (e.g. stimulation probe 16, stimulation clip 18, tilt sensor 54) , electrode harness or harnesses attached, impedance test results, myotome/EMG levels, stimulation levels, history reports, selected parameters, test results, etc... In one embodiment, set forth by way of example only, this information displayed on a main monitoring screen may include, but is not necessarily limited to the following components as set forth in Table 6:

**Table 6**

| **Screen Component** | **Description** |
|---|---|
| Patient Image/ Electrode layout | An image of the human body or relevant portion thereof showing the electrode placement on the body, with labeled channel number tabs on each side (1-4 on the left and right). Left and right labels will show the patient orientation. The channel number tabs may be highlighted or colored depending on the specific function being performed. |
| Myotome & Level Names | A label to indicate the Myotome name and corresponding Spinal Level(s) associated with the channel of interest. |
| Test Menu | A hideable menu bar for selecting between the available functional modes. |
| Device Bar | A hideable bar displaying icons and/or names of devices connected to the patient module. |
| Display Area | Shows procedure-specific information including stimulation results. |
| Color Indication | Enhances stimulation results with a color display of green, yellow, or red corresponding to the relative safety level determined by the system. |
| Stimulation Bar | A graphical stimulation indicator depicting the present stimulation status (i.e. on or off and stimulation current level), as well as providing for starting and stopping stimulation |
| Event Bar | A hideable bar that shows the last up to a selected number of previous stimulation results, provides for annotation of results, and a chat dialogue box for communicating with remote participants. |
| EMG waveforms | EMG waveforms may be optionally displayed on screen along with the stimulation results. |

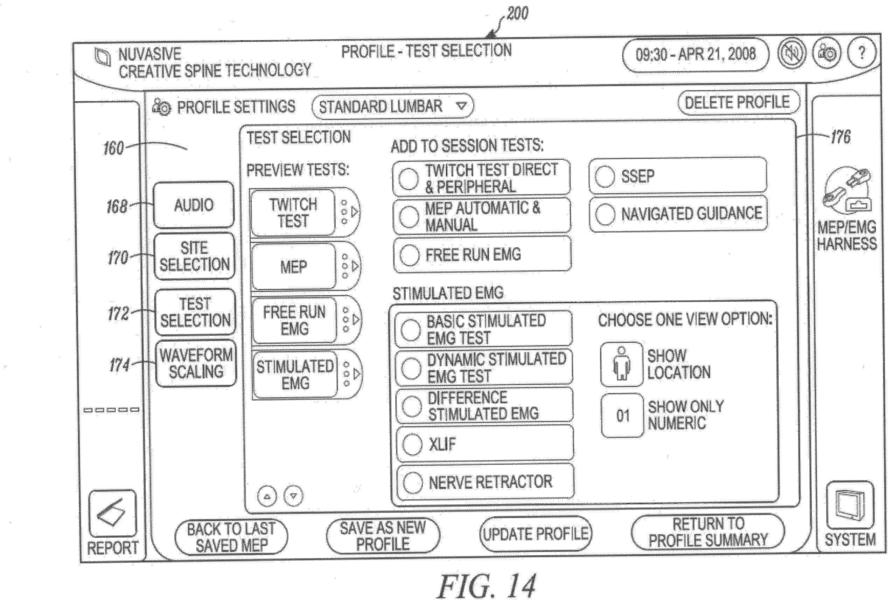

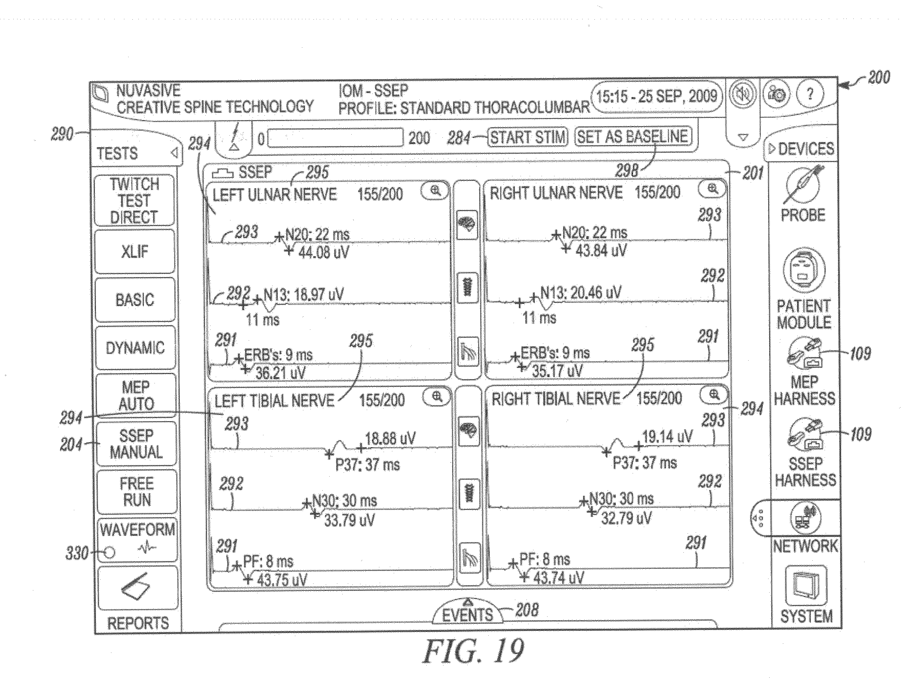

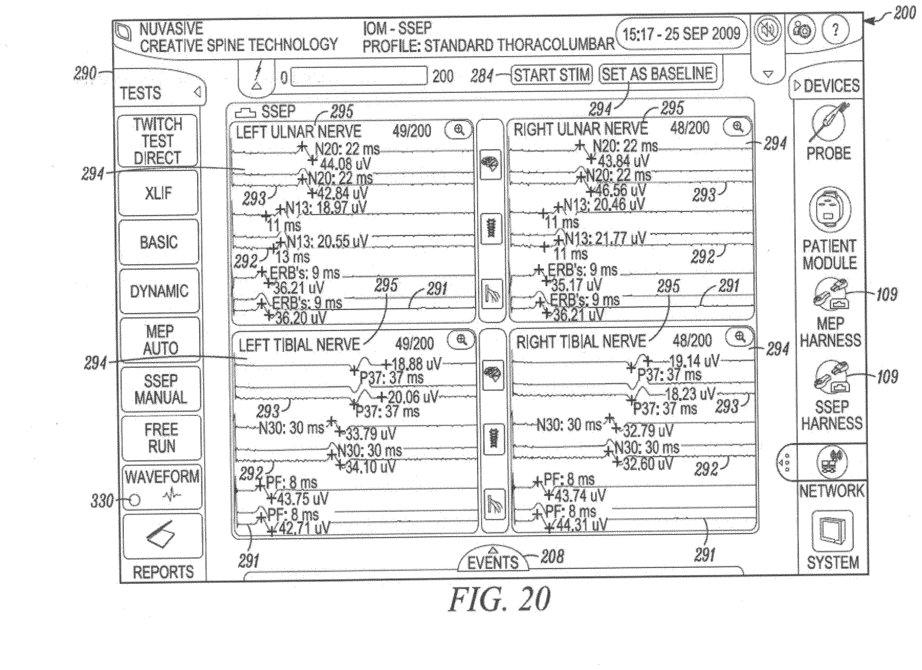

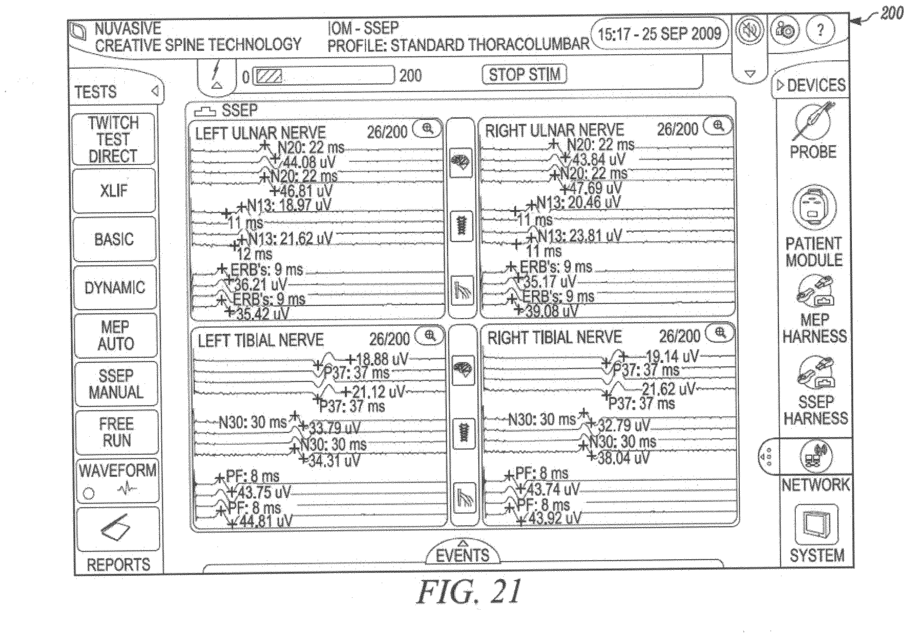

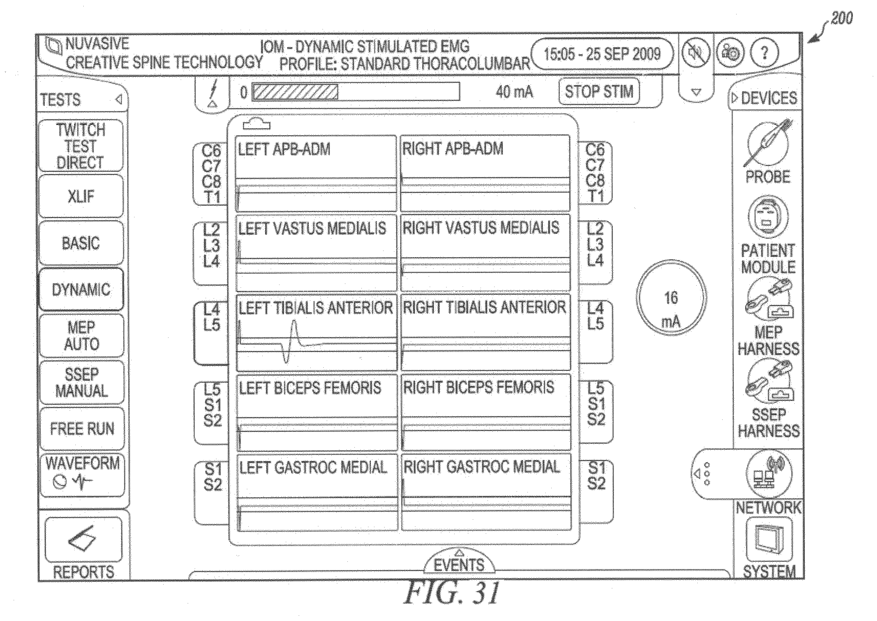

From a profile setting window 160, illustrated by way of example only in Fig. 14, custom profiles can be created and saved. Beginning with one of the standard profiles, parameters may be altered by selecting one of the audio 168, site selection 170, test selection 172, and waveform scaling 174 buttons and making the changes until the desired parameters are set. By way of example only, profiles may be generated and saved for particular procedures (e.g. ACDF, XLIF, and Decompression), particular individuals, and combinations thereof. Clicking on each button will display the parameter options specific to the selected button in a parameter window 176. The parameter options for the Test Selection Window are illustrated by way of example in Fig. 14. By way of example only, by selecting the Test Selection button, session tests may be added and viewing options may be changed. From within the test selection area, function specific parameters for all available test functions (based on site selection, available devices, etc...) may be accessed and set according to need. One option that is available for multiple functions under the test selection button is the ability to select from three different viewing options. The user may choose to see results displayed in numeric form, on a body panel, and on a label that reflects the labels associated with each electrode, or any combination of the three. The user may also choose to see the actual waveforms. Selecting the Waveform Scaling button 174 allows the user to adjust the scale on which waveforms are displayed. By selecting the audio button 168 both the system audio and Free Run audio may be adjusted. Selecting the site selection button 170 allows the opportunity to change from the site selected initially. Adjusting the site selection of the profile may alter the options available. By way of example, if the user changes the site selection from cervical to lumbar, the MEP function may no longer be selectable as an option. Fig. 13 is an example of a site selection screen. Figs. 19-26; 28-35 illustrates examples of the test selection tab for each of the test functions (e.g. SSEP Manual, SSEP Automatic, MEP Manual, MEP Automatic, Twitch Test, Basic Stimulated EMG, Dynamic Stimulated EMG, XLIF, Free-Run EMG, and Navigated Guidance). Profiles may be saved directly on the control unit 12 or they may be saved to a portable memory device, or uploaded onto a web-server.

Various features of the monitoring screen 200 of the GUI will now be described.

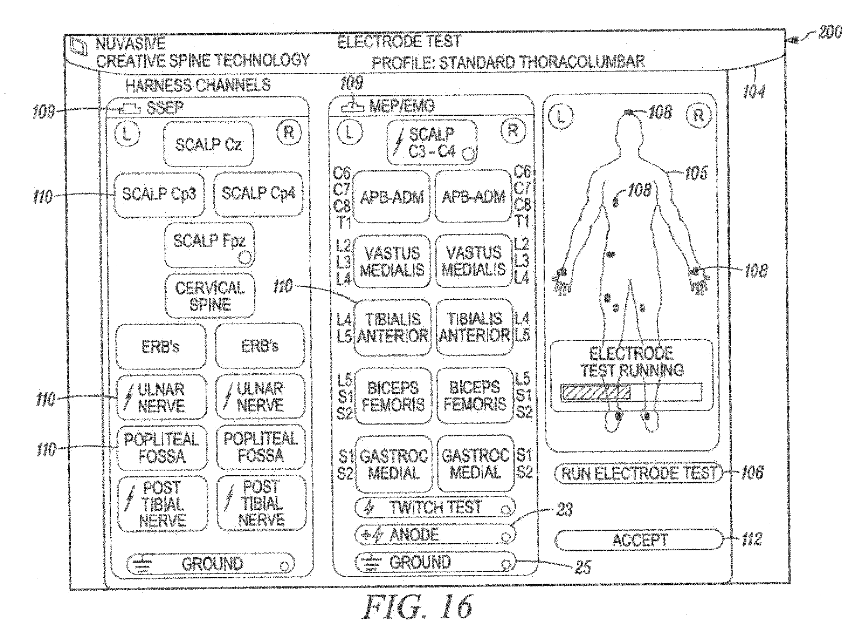

The patient module 14 is configured such that the neurophysiology system 10 may conduct an impedance test under the direction of the control unit 12 of all electrodes once the system is set up and the electrode harness is connected and applied to the patient. After choosing the appropriate spinal site upon program startup (described below), the user is automatically directed to an electrode test. Fig. 16 illustrates, by way of example only, the features of the electrode test by graphical implementations of electrode test screens according to example embodiments of the GUI. The electrode test screen 104 includes a human figure graphic 105 with electrode position indicators 108. A harness indicator 109 displays the harness or harnesses 80 that are connected to the patient module 14. For each electrode on the harness or harnesses 80 in use, including the common 25 and anode 23 electrodes, there is a corresponding channel button 110. Preferably, the common 25 and anode 23 electrodes may be independently checked for acceptable impedance. To accomplish this, the anode 23 and common 25 are both provided as dual electrodes. At least one of the anode leads on the anode electrode is reversible. During the impedance check the reversible anode lead switches to a cathode such that the impedance between the leads can be measured. When the impedance test is complete the reversible lead switches back to an anode. The channel button 110 may be labeled with the muscle name or coverage area of the corresponding electrode. Stimulation electrodes may be denoted with a symbol or other indicator, such as by way of example only, a lightning bolt in order to distinguish the recording and stimulation electrodes. Selecting a channel button 110 will disable the associated channel. Disabled channels will not be tested for impedance and they will not be monitored for responses or errors unless reactivated (e.g. by again selecting the corresponding channel button 110). Upon selection of a start button 106 (entitled "Run Electrode Test"), the system 10 tests each electrode individually to determine the impedance value. If the impedance is determined to be within acceptable limits, the channel button 110 and corresponding electrode depiction on the human figure 108 turn green. If the impedance value for any electrode is not determined to be acceptable, the associated channel button 110 and electrode depiction turn red, alerting the user. Once the test is complete, selecting the Accept button 112 will open the main monitoring screen of system 10.

The functions performed by the neuromonitoring system 10 may include, but are not necessarily limited to, the Twitch Test, Free-run EMG, Basic Stimulated EMG, Dynamic Stimulated EMG, XLIF^{®}, Nerve Retractor, MEP Manual, MEP Automatic, and SSEP Manual, SSEP Automatic, and Navigated Guidance modes, all of which will be described briefly below. The Twitch Test mode is designed to assess the neuromuscular pathway via the so-called "train-of-four test" to ensure the neuromuscular pathway is free from muscle relaxants prior to performing neurophysiology-based testing, such as bone integrity (e.g. pedicle) testing, nerve detection, and nerve retraction. This is described in greater detail within PCT Patent App. No. PCT/US2005/036089, entitled "System and Methods for Assessing the Neuromuscular Pathway Prior to Nerve Testing," filed October 7, 2005. The Basic Stimulated EMG Dynamic Stimulated EMG tests are designed to assess the integrity (e.g. pedicle) testing, nerve detection, and nerve retraction. This is described in greater detail within PCT Patent App. No. PCT/US2005/036089, entitled "System and Methods for Assessing the Neuromuscular Pathway Prior to Nerve Testing," filed October 7, 2005. The Basic Stimulated EMG Dynamic Stimulated EMG tests are designed to assess the integrity of bone (e.g. pedicle) during all aspects of pilot hole formation (e.g., via an awl), pilot hole preparation (e.g. via a tap), and screw introduction (during and after). These modes are described in greater detail in PCT Patent App. No. PCT/US02/35047 entitled "System and Methods for Performing Percutaneous Pedicle Integrity Assessments," filed on October 30, 2002, and PCT Patent App. No. PCT/US2004/025550, entitled "System and Methods for Performing Dynamic Pedicle Integrity Assessments," filed on August 5, 2004. The XLIF mode is designed to detect the presence of nerves during the use of the various surgical access instruments of the neuromonitoring system 10, including the pedicle access needle 26, k-wire 42, dilator 44, and retractor assembly 70. This mode is described in greater detail within PCT Patent App. No. PCT/US2002/22247, entitled "System and Methods for Determining Nerve Proximity, Direction, and Pathology During Surgery," filed on July 11, 2002. The Nerve Retractor mode is designed to assess the health or pathology of a nerve before, during, and after retraction of the nerve during a surgical procedure. This mode is described in greater detail within PCT Patent App. No. PCT/US2002/30617, entitled "System and Methods for Performing Surgical Procedures and Assessments," filed on Sept. 25, 2002. The MEP Auto and MEP Manual modes are designed to test the motor pathway to detect potential damage to the spinal cord by stimulating the motor cortex in the brain and recording the resulting EMG response of various muscles in the upper and lower extremities. The SSEP function is designed to test the sensory pathway to detect potential damage to the spinal cord by stimulating peripheral nerves inferior to the target spinal level and recording the action potential from sensors superior to the spinal level. The MEP Auto, MEP manual, and SSEP modes are described in greater detail within PCT Patent App. No. PCT/US2006/003966, entitled "System and Methods for Performing Neurophysiologic Assessments During Spine Surgery," filed on February 2, 2006. The Navigated Guidance function is designed to facilitate the safe and reproducible use of surgical instruments and/or implants by providing the ability to determine the optimal or desired trajectory for surgical instruments and/or implants and monitor the trajectory of surgical instruments and/or implants during surgery. This mode is described in greater detail within PCT Patent App. No. PCT/US2007/11962, entitled "Surgical Trajectory Monitoring System and Related Methods," filed on July 30, 2007, and PCT Patent App. No. PCT/US2008/12121. These functions will be explained now in brief detail.

The neuromonitoring system 10 performs assessments of spinal cord health using one or more of MEP Auto, MEP Manual, SSEP Auto, and SSEP manual modes.

In the SSEP modes, the neuromonitoring system 10 stimulates peripheral sensory nerves that exit the spinal cord below the level of surgery and then measures the electrical action potential from electrodes located on the nervous system superior to the surgical target site. Recording sites below the applicable target site are also preferably monitored as a positive control measure to ensure variances from normal or expected results are not due to problems with the stimulation signal deliver (e.g. misplaced stimulation electrode, inadequate stimulation signal parameters, etc.). To accomplish this, stimulation electrodes 22 may be placed on the skin over the desired peripheral nerve (such as by way of example only, the left and right Posterior Tibial nerve and/or the left and right Ulnar nerve) and recording electrodes 24 are positioned on the recording sites (such as, by way of example only, C2 vertebra, Cp3 scalp, Cp4 scalp, Erb's point, Popliteal Fossa) and stimulation signals are delivered from the patient module 14.

Damage in the spinal cord may disrupt the transmission of the signal up along the spinothalamic pathway through the spinal cord resulting in a weakened, delayed, or absent signal at the recording sites superior to the surgery location (e.g. cortical and subcortical sites). To check for these occurrences, the system 10 monitors the amplitude and latency of the evoked signal response. According to one embodiment, the system 10 may perform SSEP in either of two modes: Automatic mode and Manual mode. In SSEP Auto mode, the system 10 compares the difference between the amplitude and latency of the signal response vs. the amplitude and latency of a baseline signal response. The difference is compared against predetermined "safe" and "unsafe" levels and the results are displayed on display 34. According to one embodiment, the system may determine safe and unsafe levels based on each of the amplitude and latency values for each of the cortical and subcortical sites individually, for each stimulation channel. That is, if either of the subcortical and cortical amplitudes decrease by a predetermined level, or either of the subcortical and cortical latency values increase by a predetermined level, the system may issue a warning.

By way of example, the alert may comprise a Red, Yellow, Green type warning associated with the applicable channel wherein Red indicates that at least one of the determined values falls within the unsafe level, the color green may indicate that all of the values fall within the safe level, and the color yellow may indicate that at least one of the values falls between the safe and unsafe levels. To generate more information, the system 10 may analyze the results in combination. With this information, in addition to the Red, Yellow, and Green alerts, the system 10 may indicate possible causes for the results achieved. In SSEP Manual mode, signal response waveforms and amplitude and latency values associated with those waveforms are displayed for the user. The user then makes the comparison between a baseline and the signal response.

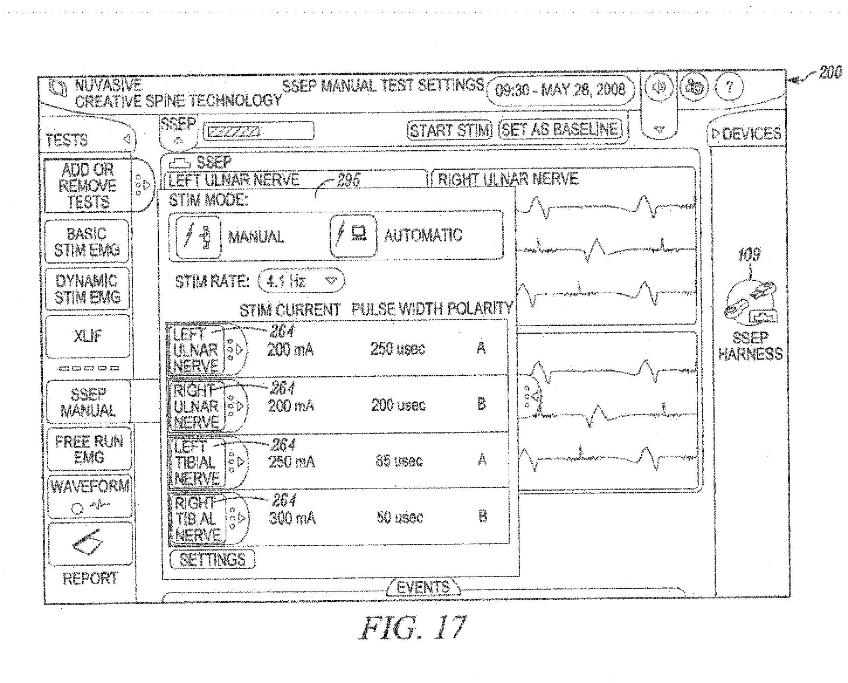

Figs. 17-22 are exemplary screen displays of the "SSEP Manual" mode according to one embodiment of the neuromonitoring system 10. Fig. 17 illustrates an intraoperative monitoring (IOM) setup screen from which various features and parameters of the SSEP Manual mode may be controlled and/or adjusted by the user as desired. Using this screen, the user has the opportunity to toggle between Manual mode and Automatic mode, select a stimulation rate, and change one or more stimulation settings (e.g. stimulation current, pulse width, and polarity) for each stimulation target site (e.g. left ulnar nerve, right ulnar nerve, left tibial nerve, and right tibial nerve). By way of example only, the user may change one or more stimulation settings of each peripheral nerve by first selecting one of the stimulation site tabs 264.

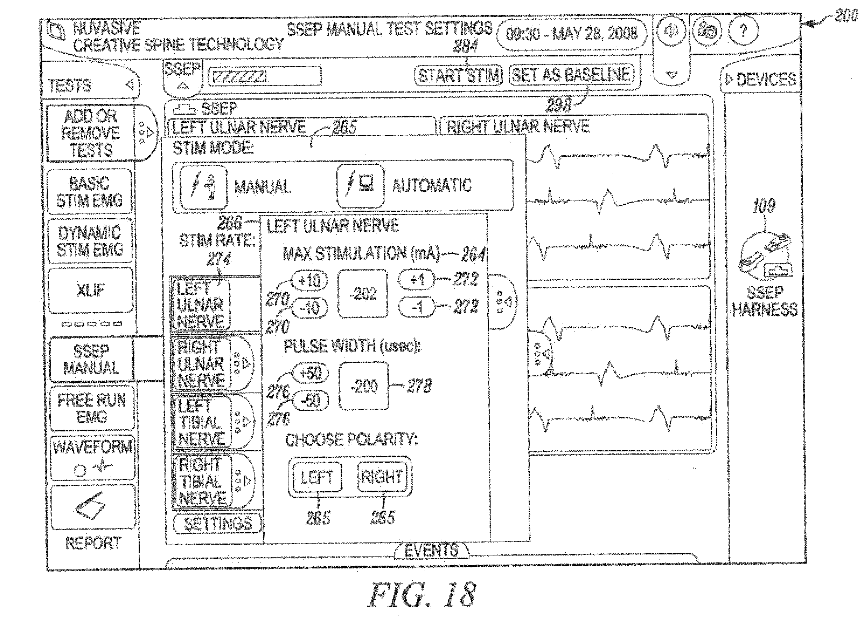

Selecting one of the stimulation site tabs 264 will open a control window 265, seen in Fig. 18, from which various parameters of the SSPE manual test may be adjusted according to user preference. By way of example only, FIG. 18 is an illustration of an onscreen display for the SSEP manual test settings of the left ulnar nerve stimulation site. The highlighted "Left Ulnar Nerve" stimulation site tab 264 and the pop-up window title 266 indicate that adjusting any of the settings will alter the stimulation signal delivered to the left ulnar nerve. Multiple adjustment buttons are used to set the parameters of the stimulation signal. According to one example, the stimulation rate may be selected from a range between 2.2 and 6.2 Hz, with a default value of 4.7 Hz. The amplitude setting may be increased or decreased in increments of 10mA using the amplitude selection buttons 270 labeled (by way of example only) "+10" and "-10". More precise amplitude selections may be made by increasing or decreasing the amplitude in increments of 1mA using the amplitude selection buttons 272 labeled (by way of example only) "+1" and "-1". According to one example, the amplitude may be selected from a range of 1 to 100 mA with a default value of 10mA. The selected amplitude setting is displayed in box 274. The pulse width setting may be increased or decreased in increments of 50 psec using the width selection buttons 276 labeled "+50" and "-50". According to one example, the pulse width may be selected from a range of 50 to 300 psec, with a default value of 200 psec. The precise pulse width setting 278 is indicated in box 278. Polarity controls 280 may be used to set the desired polarity of the stimulation signal. SSEP stimulation may be initiated at the selected stimulation settings by pressing the SSEP stimulation start button 284 labeled (by way of example only) "Start Stim." Although stimulation settings adjustments are discussed with respect to the left ulnar nerve, it will be appreciated that stimulation adjustments may be applied to the other stimulation sites, including but not limited to the right ulnar nerve, and left and right tibial nerve. AlternativleyAlternatively, as described below, the system 10 may utilize an automated selection process to quickly determine the optimal stimulation parameters for each stimulation channel.

In order to monitor the health of the spinal cord with SSEP, the user must be able to determine if the responses to the stimulation signal are changing. To monitor for this change a baseline is determined, preferably during set-up. This can be accomplished simply by selecting the "set as baseline" button 298 next to the "start stim" button 284 on the setting screen illustrated in Fig. 18. Having determined a baseline recording for each stimulation site, subsequent monitoring may be performed as desired throughout the procedure and recovery period to obtain updated amplitude and latency measurements.

Fig. 19 depicts an exemplary screen display for Manual mode of the SSEP monitoring function. A mode indicator tab 290 on the test menu 204 indicates that "SSEP Manual" is the selected mode. The center result area 201 is divided into four sub areas or channel windows 294, each one dedicated to displaying the signal response waveforms for one of the stimulation nerve sites. The channel windows 294 depict information including the nerve stimulation site 295, and waveform waterfalls for each of the recording locations 291-293. For each stimulated nerve site, the system 10 displays three signal response waveforms, representing the measurements made at three different recording sites. By way of example only, the three recording sites are a peripheral 291 (from a peripheral nerve proximal to the stimulation nerve), subcortical 292 (spine), and cortical 293 (scalp), as indicated for example in Table 5 above. Each section may be associated with a pictorial icon, illustrating the neural/skeletal structure. Although SSEP stimulation and recording is discussed with respect to the nerve stimulation site and the recording sites discussed above, it will be appreciated that SSEP stimulation may be applied to any number of peripheral sensory nerves and the recording sites may be located anywhere along the nervous system superior to the spinal level at risk during the procedure.

During SSEP modes (auto and manual), a single waveform response is generated for each stimulation signal run (for each stimulation channel). The waveforms are arranged with stimulation on the extreme left and time increasing to the right. By way of example, the waveforms are captured in a 100 ms window following stimulation. The stimulation signal run is comprised of a predefined number of stimulation pulses firing at the selected stimulation frequency. By way of example only, the stimulation signal may include 300 pulses at a frequency of 4.7Hz. A 100 ms window of data is acquired on each of three SSEP recording channels: cortical, subcortical, and peripheral. With each successive stimulation on the same channel during a stimulation run, the three acquired waveforms are summed and averaged with the prior waveforms during the same stimulation run for the purpose of filtering out asynchronous events such that only the synchronous evoked response remains after a sufficient number of pulses. Thus, the final waveform displayed by the system 10 represents an averaging of the entire set (e.g. 300) of responses detected.

With each subsequent stimulation run, waveforms are drawn slightly lower each time, as depicted in Figs. 19-21, until a total of four waveforms are showing. After more than four stimulation runs, the baseline waveform is retained, as well as the waveforms from the previous four stimulation runs. Older waveforms are removed from the waveform display. According to one embodiment, different colors may be used to represent the different waveforms. For example, the baseline waveforms may be colored purple, the last stimulation run may be colored white, the next-to-last stimulation run may be colored medium gray, and the earliest of the remaining stimulation runs may be colored dark gray.

According to one example, the baseline and the latest waveforms may have markers 314, 316 placed indicating latency and amplitude values associated with the waveform. The latency is defined as the time from stimulation to the first (earliest) marker. There is one "N" 314 and one "P" 316 marker for each waveform. The N marker is defined as the maximum average sample value within a window and the P value is defined as the minimum average sample value within the window. The markers may comprise cross consisting of a horizontal and a vertical line in the same color as the waveform. Associated with each marker is a text label 317 indicating the value at the marker. The earlier of the two markers is labeled with the latency (e.g. 22.3 ms). The latter of the two markers is labeled with the amplitude (e.g. 4.2 uV). The amplitude is defined as the difference in microvolts between average sample values at the markers. The latency is defined as the time from stimulation to the first (earliest) marker. Preferably, the markers are placed automatically by the system 10 (in both auto and manual modes). In manual mode, the user may select to place (and or move) markers manually.

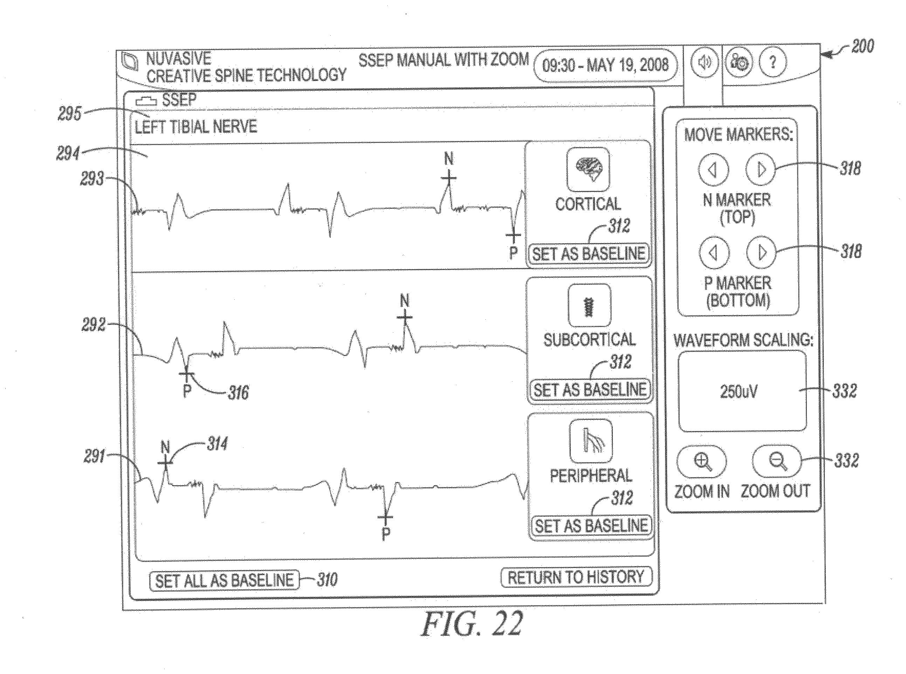

Further selecting one of the channel windows 294 will zoom in on the waveforms contained in that window 294. Fig. 22 is an example illustration of the zoom view achieved by selecting one of the channel windows 294. The zoom view includes waveforms 291-293, the baseline waveform, markers 314 and 316, and controls for moving markers 318 and waveform scaling 332. Only the latest waveform is shown. The "Set All as Baseline" button 310 will allow the user to set (or change) all three recorded waveforms as the baselines. Additionally, baselines may be set (or changed) individually by pressing the individual "Set as Baseline" buttons 312. Furthermore, the user may also move the N marker 314 and P markers 316 to establish new measurement points if desired. Direction control arrows 318 may be selected to move the N and P markers to the desired new locations. Alternatively, the user may touch and drag the marker 314, 316 to the new location. Utilizing the waveform controls 332 the user may zoom in and out on the recorded waveform.

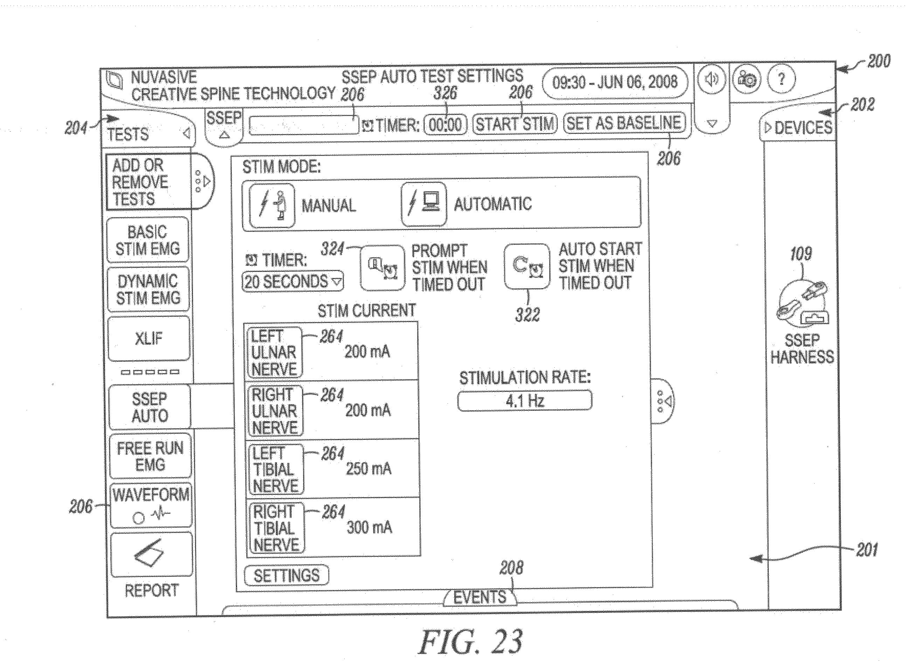

Referencing Figs. 23-26, Automatic SSEP mode functions similar to Manual SSEP mode except that the system 10 determines the amplitude and latency values and alerts the user if the values deviate. Fig. 23 shows, by way of example only, an exemplary setup screen for the SSEP Automatic mode. In similar fashion to the setup screen previously described for the SSEP Manual mode, the user may toggle between Manual mode and Automatic mode, select a stimulation rate, and change one or more stimulation settings. By way of example only, the user may change one or more stimulation settings of each peripheral nerve by first selecting one of the stimulation site tabs 264, as described above with reference to Manual mode and Fig. 18. According to one example, the stimulation rate may be selected from a range between 2.2 and 6.2 Hz, with a default value of 4.7 Hz, the amplitude may be selected from a range of 1 to 100 mA, with a default value of 10mA, the pulse width may be selected from a range of 50 to 300 psec, with a default value of 200 psec.

In Automatic mode, the surgical system 10 also includes a timer function which can be controlled from the setup screen. Using the timer drop down menu 326, the user may set and/or change a time interval for the timer application. There are two separate options of the timer function: (1) an automatic stimulation on time out which can be selected by pressing the auto start button 322 labeled (by way of example only) "Auto Start Stim when timed out"; and (2) a prompted stimulation reminder on time out which can be selected by pressing the prompt stimulation button 324 labeled (by way of example only) "Prompt Stim when timed out". After each SSEP monitoring episode, the system 10 will initiate a timer corresponding to the selected time interval and, when the time has elapsed, the system will either automatically perform the SSEP stimulation or a stimulation reminder will be activated, depending on the selected option. The stimulation reminder may include, by way of example only, any one of, or combination of, an audible tone, voice recording, screen flash, pop up window, scrolling message, or any other such alert to remind the user to test SSEP again. It is also contemplated that the timer function described may be implemented in SSEP Manual mode.

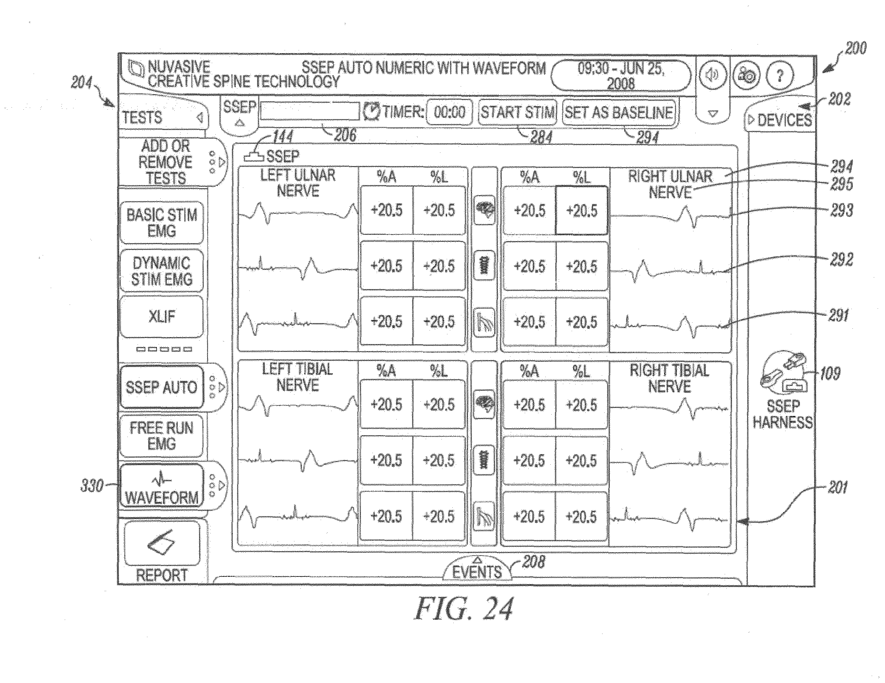

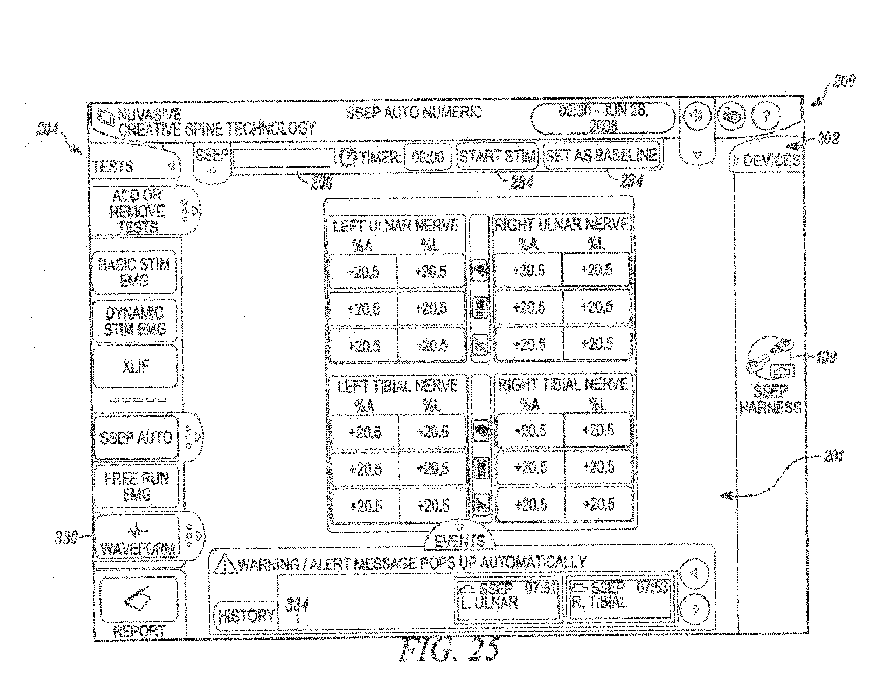

Figs. 24-26 depict exemplary onscreen displays for Automatic mode of the SSEP function. According to one embodiment, the user may select to view a screen with only alpha-numeric information (Fig. 25) and one with alpha-numeric information and recorded waveforms (Fig. 24). A mode indicator tab 290 indicates that "SSEP Auto" is the selected mode. A waveform selection tab 330 allows the user to select whether waveforms will be displayed with the alpha-numeric results. In similar fashion to the onscreen displays previously described for the SSEP Manual mode, the system 10 includes a channel window 294 for each nerve stimulation site. The channel window 294 may display information including the nerve stimulation site 295, waveform recordings, and associated recording locations 291-293 (peripheral, sub cortical, and cortical) and the percentage change between the baseline and amplitude measurements and the baseline and latency measurements. By way of example only, each channel window 294 may optionally also show the baseline waveform and latest waveform for each recording site. In the event the system 10 detects a significant decrease in amplitude or an increase in latency, the associated window may preferably be highlighted with a predetermined color (e.g. red) to indicate the potential danger to the surgeon. Preferably, the stimulation results are displayed to the surgeon along with a color code so that the user may easily comprehend the danger and corrective measures may be taken to avoid or mitigate such danger. This may for example, more readily permit SSEP monitoring results to be interpreted by the surgeon or assistant without requiring dedicated neuromonitoring personnel. By way of example only, red is used when the decrease in amplitude or increase in latency is within a predetermined unsafe level. Green indicates that the measured increase or decrease is within a predetermined safe level. Yellow is used for measurements that are between the predetermined unsafe and safe levels. By way of example only, the system 10 may also notify the user of potential danger through the use of a warning message 334. Although the warning message is in the form of a pop-up window, it will be appreciated that the warning may be communicated to the user by any one of, or combination of, an audible tone, voice recording, screen flash, scrolling message, or any other such alert to notify the user of potential danger

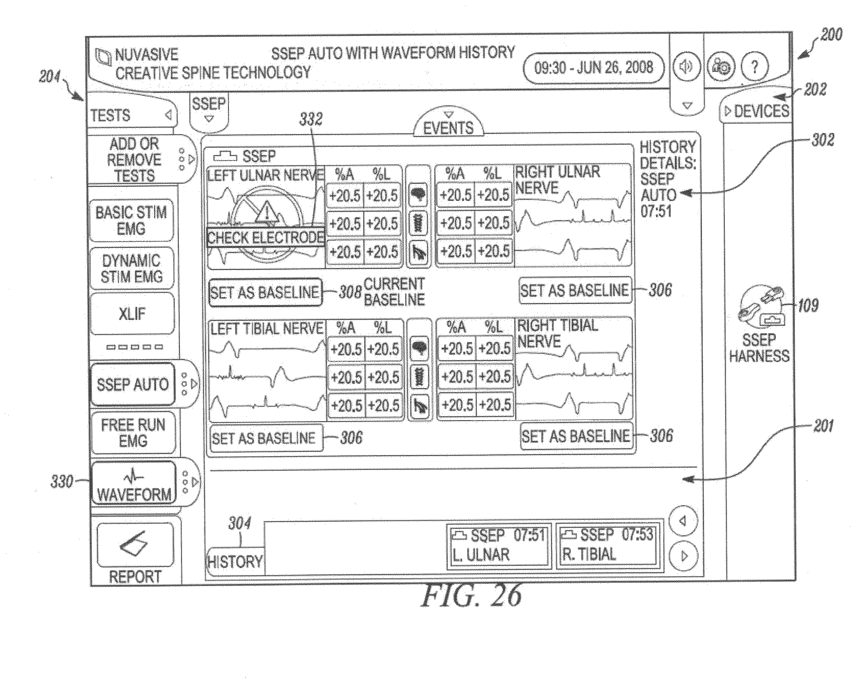

With reference to Fig. 26 at any time during the procedure, a prior stimulation run may be selected for review. This may be accomplished by, for example, by opening the event bar 208 and selecting the desired event. Details from the event are shown with the historical details denoted on the right side of the menu screen 302 and waveforms shown in the center result screen. Again, the user may chose to reset baselines for one or more nerve stimulation sites by pressing the appropriate "Set As Baseline" button 306. In the example shown, the system 10 illustrates the waveform history at the 07:51 minute mark which is denoted on the right side of the menu screen 302. Prior waveform histories are saved by the surgical system 10 and stored in the waveform history toolbar 304. The describe Though described only in relation to the SSEP Auto function, it will be appreciated that the same features may be accessed from SSEP Manual mode[[,]]. T[[t]]he user may choose to set a recorded stimulation measurement as the baseline for each nerve stimulation site by pressing the "Set As Baseline" button 306. By way of example only, the system 10 will inform the user if the applicable event is already the current baseline with a "Current Baseline" notification 308.

In addition to alerting the user to any changes in the amplitude and/or latency of the SSEP signal response, it is further contemplated that the neuromonitoring system 10 may assess the data from all the recording sites to interpret possible causes for changes in the SSEP response. Based on that information, the program may suggest potential reasons for the change. Furthermore, it may suggest potential actions to be taken to avoid danger. It is still further contemplated that the neurophysiology system 10 may be communicatively linked with other equipment in the operating room, such as for example, anesthesia monitoring equipment. Data from this other equipment may be considered by the program to generate more accuracy and or better suggestions. By way of example only, Table 7 illustrates the SSEP illustrates various warnings that may be associated with particular SSEP results and result combination, and show to the user. For example, if in response to stimulation of the left ulnar nerve, the peripheral response from Erb's Point showed no change in amplitude or latency, the subcortical response showed a decrease in amplitude, and the cortical response showed a decrease in amplitude, the event box 206 (shown in Fig. 25) would show either a yellow or a red indicator as well as the text "Possible mechanical insult. Possible spinal cord ischemia." By way of another example, if there is a decreased amplitude or absent response in all peripheral, subcortical, and cortical recording sites, the system may show a "Check Electrode" warning 332 (Fig. 26). With this date, and the particular circumstances leading to the result (e.g. what surgical maneuver resulted in the warning, etc.) the user may be better equipped to determine the most prudent course of action.

**Table 7:**

| Neurophysiologic Event | Audiovisual Alert (Color) | SSEP Expert Text |
|---|---|---|
| Cortical amplitude decrease: | Green | No Warning |
| 0-25% from baseline: | | |
| Cortical amplitude decrease: 26-49% from baseline | Yellow | "Some anesthetic agents may reduce the cortical response amplitude." |
| Cortical amplitude decrease: 50%-99% from baseline | Red | "Some anesthetic agents may reduce the cortical response amplitude." |
| Cortical amplitude decrease: 100% from baseline | Red | "Possible cortical ischemia." |
| Cortical latency increase: 0-5% from baseline | Green | No Warning |
| Cortical latency increase: 6-9% from baseline | Yellow | "Some anesthetic agents may increase the cortical response latency. Possible cortical ischemia." |
| Cortical latency increase : 10% or greater from baseline | Red | "Some anesthetic agents may increase the cortical response latency. Possible cortical ischemia." |
| Cortical response absent: | Red | "Some anesthetic agents may cause the cortical response to be absent. Possible cortical ischemia." |
| Subcortical amplitude decrease: 0%-25% from baseline | Green | No Warning |
| Subcortical amplitude decrease: 25%-49% from baseline | Yellow | "Possible muscle activity artifact. Possible cervical recording electrode issue." |
| Subcortical amplitude decrease: 50-99% from baseline or absent | Red | "Possible muscle activity artifact. Possible cervical recording issue." |
| 50% amplitude decrease, 10% latency increase in both cortical and subcortical responses, or absence in both cortical and subcortical responses: | Red | "Possible mechanical insult. Possible spinal cord ischemia." |
| Peripheral amplitude decrease: greater than 50% or absent | Red | "Possible peripheral recording electrode issue." |
| Peripheral (Erb's Point) amplitude decrease: 0-25% from baseline | Green | No Warning (left or right) |
| Peripheral (Erb's Point) amplitude decrease: | Yellow | "Possible peripheral recording electrode issue (Left Erb's Point)." |
| 26-49% from baseline | | "Possible peripheral recording electrode issue (Right Erb's Point)." |
| Peripheral (Erb's Point) amplitude decrease: | Red | "Possible peripheral recording electrode issue (Left Erb's Point)." |
| 50%-100% from baseline | | "Possible peripheral recording electrode issue (Right Erb's Point)." |
| Peripheral (Popliteal Fossa amplitude decrease: | Green | No Warning (left or right) |
| 0-25% from baseline | | |
| Peripheral (Popliteal Fossa) amplitude decrease: | Yellow | "Possible peripheral recording electrode issue (Left Popliteal Fossa)." |
| 26-49% from baseline | | "Possible peripheral recording electrode issue (Right Popliteal Fossa)." |
| Peripheral (Popliteal Fossa) amplitude decrease: | Red | "Possible peripheral recording electrode issue (Left Popliteal Fossa)." |
| 50%-100% from baseline | | "Possible peripheral recording electrode issue (Right Popliteal Fossa)." |
| Peripheral (Erb's Point) latency increase: | Green | No Warning (left or right) |
| 0-5% from baseline | | |
| Peripheral (Erb's Point) latency increase: 6-9% from baseline | Yellow | No Warning (left or right) |
| Peripheral (Erb's Point) latency increase: | Red | No Warning (left or right) |
| 10% or greater from baseline | | |
| Peripheral (Popliteal Fossa) latency increase: | Green | No Warning (left or right |
| 0-5% from baseline | | |
| Peripheral (Popliteal Fossa) latency increase: | Yellow | No Warning (left or right) |
| 6-9% from baseline | | |
| Peripheral (Popliteal Fossa) latency increase: | Red | No Warning (left or right) |
| 10% or greater from baseline | | |
| Peripheral (Popliteal Fossa) and subcortical amplitude decrease: 0-25% from baseline | Green | Possible muscle activity artifact. |
| | | Possible cervical recording electrode issue. (left or right) |
| Peripheral (Popliteal Fossa) and subcortical amplitude decrease: 26%-100% from baseline | Yellow/ Red | "Possible cervical muscle activity artifact. Possible cervical recording electrode issue. Possible muscle activity artifact (posterior tibial nerve)." (left or right) |
| Peripheral (Erb's Point) and subcortical amplitude decrease: 0-25% from baseline | Green | "Possible muscle activity artifact. |
| | | Possible cervical recording electrode issue." (left or right) |
| Peripheral (Erb's Point) and subcortical amplitude decrease: 26-99% from baseline | Yellow/ Red | "Possible cervical muscle activity artifact. Possible cervical recording electrode issue. Possible muscle activity artifact (median nerve)." (left or right) |
| Decreased amplitude or absent response in all, peripheral (left Erb's point), subcortical, and cortical: | Yellow/ Red | "Possible stimulating electrode issue. (left wrist)." |
| Decreased amplitude or absent in all, peripheral (right Erb's point), subcortical, and cortical: | Yellow/ Red | "Possible stimulating electrode issue (right wrist)." |
| Decreased amplitude or absent response in all peripheral (left Popliteal Fossa), subcortical, and cortical: | Yellow/ Red | "Possible stimulating electrode issue (left ankle)." |
| Decreased amplitude or absent response in all peripheral (right Popliteal | Yellow/ Red | Possible stimulating electrode issue (right ankle) |
| Fossa), subcortical, and cortical | | |
| Increased latency or decreased amplitude in all, peripheral, subcortical, and cortical: | Yellow/ Red | "Possible systemic change (hypotension, hypothermia, hyperthermia). Possible peripheral nerve ischemia." (left or right) (posterior tibial or ulnar nerve) |

As mentioned above, the system 10 may employ an automated tests to quickly select the optimal stimulus parameters for conducting SSEP testing on each active stimulation channel. This can be done according to any number of algorithms that automatically adjust various parameters until a combination resulting in the most desirable result is achieved. By way of example, the system 10 may utilize an algorithm similar to the hunting algorithm described below for finding Iₜₕᵣₑₛₕ for EMG and MEP modalities. According to this example, the desired stimulation parameters are determined by first finding the lowest Iₜₕᵣₑₛₕ (that is the lowest stimulation signal intensity that results in a waveform having a predetermined amplitude, Vₜₕᵣₑₛₕ) for each stimulation site (e.g., left posterior tibial nerve (LPTN), right posterior tibial nerve (RPTN), left ulnar nerve (LUN), and right ulnar nerve (RUN)). By way of example only, to determine the Iₜₕᵣₑₛₕ for a LPTN, using polarity A (cathode proximal to the surgical site), an initial, predetermined stimulus intensity is applied transcutaneously to the left PTN stimulation site. If no response is obtained from recording electrodes at the left popliteal fossa with a Vₚₚ greater or equal to Vₜₕᵣₑₛₕ, polarity B is used (anode proximal to the surgical site), and the same stimulus intensity is applied. If no response is obtained at the first stimulus level for either polarity, the polarity is again switched and the stimulation intensity is doubled. Thus, using polarity A, a second stimulus intensity is applied. If there is no response recorded from the left popliteal fossa, the polarity is reversed and a stimulus of the same second intensity is applied. If there is still no response that recruits (results in a Vₚₚ at or above Vₜₕᵣₑₛₕ), the stimulus intensity is again doubled until there is an evoked potential with a Vₚₚ greater or equal to Vₜₕᵣₑₛₕ. The polarity setting from which the first evoked potential recorded in the left popliteal fossa that achieves Vₜₕᵣₑₛₕ, is set as the polarity for this stimulation site. The first stimulation intensity to achieve Vₜₕᵣₑₛₕ and the immediately previous stimulation intensity form an initial bracket.

After the threshold current Iₜₕᵣₑₛₕ has been bracketed, the initial bracket is successively reduced via bisection to a predetermined width. This is accomplished by applying a first bisection stimulation current that bisects (i.e. forms the midpoint of) the initial bracket. If this first bisection stimulation current recruits, the bracket is reduced to the lower half of the initial bracket. If this first bisection stimulation current does not recruit, the bracket is reduced to the upper half of the initial bracket. This process is continued for each successive bracket until Iₜₕᵣₑₛₕ is bracketed by stimulation currents separated by the predetermined width. Once Iₜₕᵣₑₛₕ is determined for a particular stimulation channel, the stimulus intensity is set as the value 20% greater than the detected threshold. This is repeated for each stimulation channel until the optimal stimulation signal is set for each. The optimal stimulation signal may be determined for each stimulation channel in sequence, or, simultaneously (by proceeding in similar fashion to the multi channel threshold detection algorithm described below. The determined stimulation values will then preferably be used throughout the monitoring procedure.

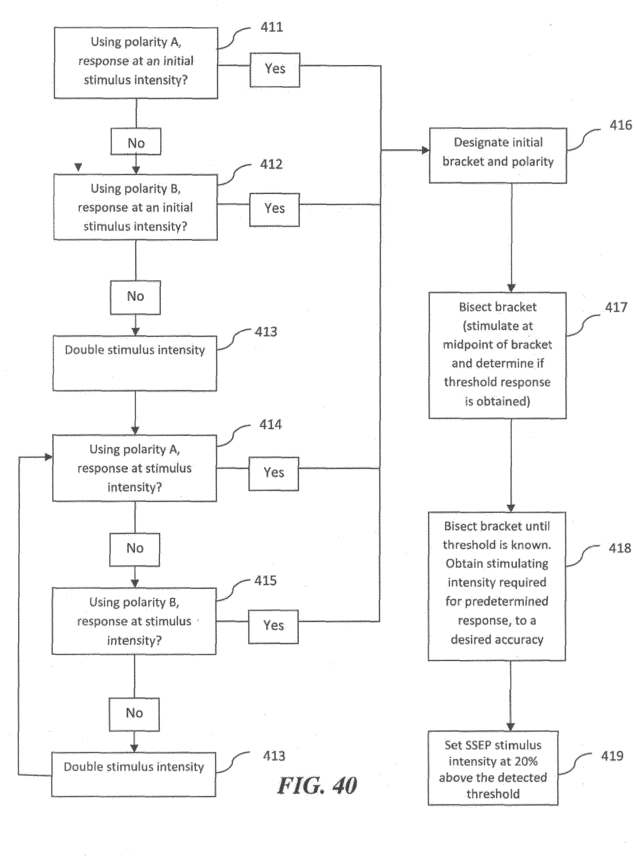

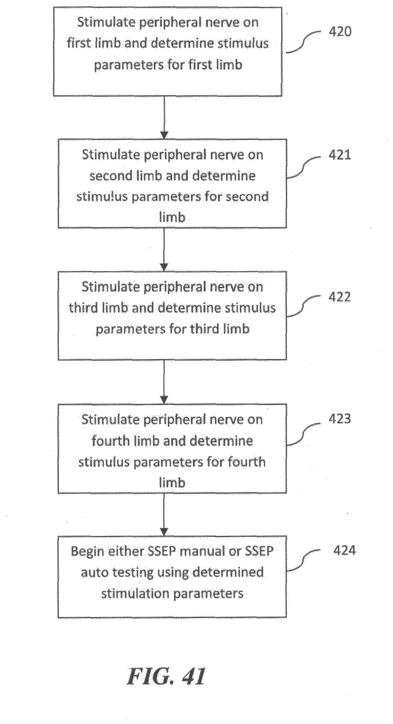

The threshold hunting algorithm for optimizing SSEP stimulation parameter is further described with reference to Figs. 40-41. Fig. 40 illustrates (in flowchart form) a method by which the stimulus intensity algorithm quickly searches for the optimal stimulation parameters. The algorithm first stimulates at an initial stimulation intensity using polarity A, and determines whether this results in an Irecruit (step 411). If the algorithm determines that there has been no recruitment, the algorithm reverses the direction of the polarity and stimulates at the same initial stimulation intensity using polarity B and determines whether this results in an Irecruit (step 412). If the algorithm determines that there has been no recruitment[[f]], the algorithm moves to step 413 and doubles the stimulation intensity. At step 414, using polarity A, the algorithm stimulates at the second intensity and determines if this is an Irecruit (step 414). If the answer is no, the algorithm proceeds to step 415, reverses to polarity B, and stimulates at the second intensity. If the answer is still no, then step 413 is repeated and the stimulus intensity is doubled again. If at any point during step 411, 413, 414, or 415 the answer is yes, the algorithm designates this as the initial bracket and polarity as shown in step 416 and as previously described. The algorithm then moves to step 417 and the bracket is bisected. In other words, the stimulation is performed at the midpoint of the bracket. At step 418, the algorithm bisects the bracket until a threshold is known and the stimulating intensity required for a predetermined response is obtained to a desired accuracy. At step 419, the SSEP stimulus intensity is set at 20% above the detected threshold. Once Iₜₕᵣₑₛₕ is found for Limb 1, as shown in step 420 of FIG. 41, the algorithm begins a second step (step 421) and processes Limb 2 by mirroring steps 411-419. This same process is repeated for Limb 3(step 422) and Limb 4 (step 423). After the stimulus intensity algorithm has determined the optimal stimulus parameters, SSEP neurophysiologic testing may be commenced (step 424).

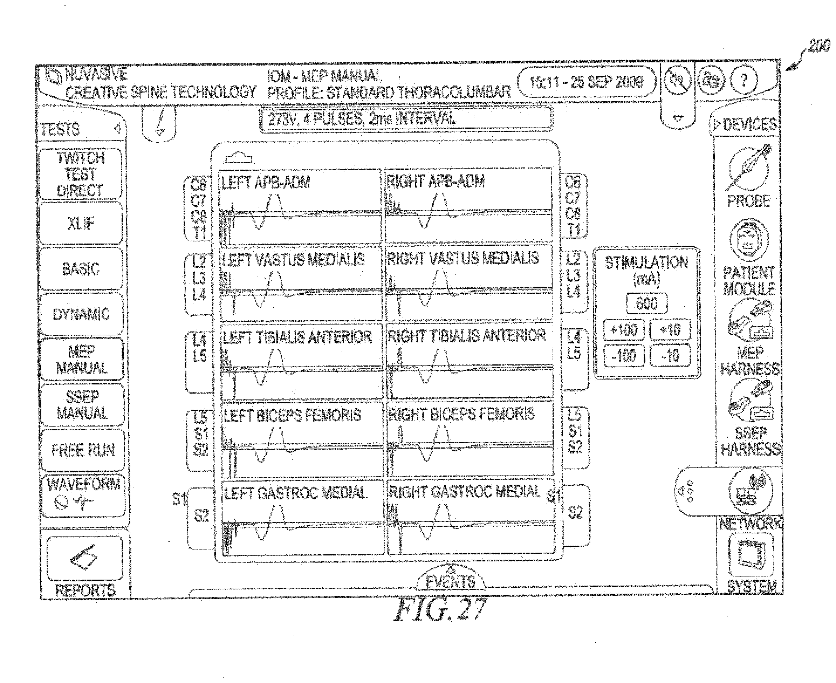

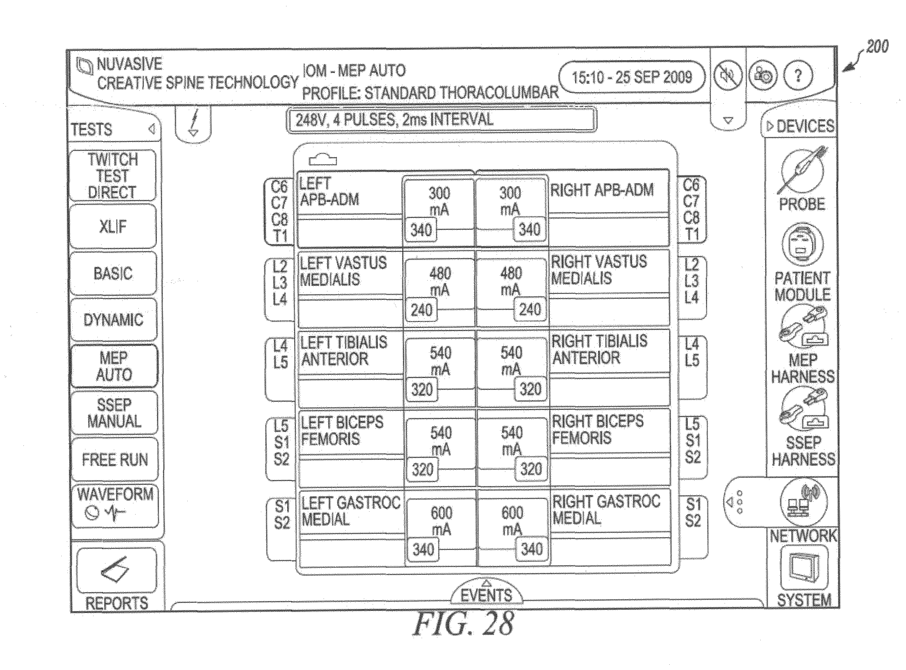

With reference to Figs. 27-39, the remaining functions of the neurophysiologic monitoring system 10 will be described in brief detail. In MEP modes, stimulation signals are delivered to the Motor Cortex via patient module 14 and resulting responses are detected from various muscles in the upper and lower extremities. An increase in Iₜₕᵣₑₛₕ from an earlier test to a later test may indicate a degradation of spinal cord function. Likewise, the absence of a significant EMG response to a given Iₛₜᵢₘ on a channel that had previously reported a significant response to the same or lesser Iₛₜᵢₘ is also indicative of a degradation in spinal cord function. These indicators are detected by the system in the MEP modes and reported to the surgeon. In MEP Auto mode the system determines the Iₜₕᵣₑₛₕ baseline for each channel corresponding to the various monitored muscles, preferably early in the procedure, using the multi-channel algorithm described. Throughout the procedure subsequent tests may be conducted to again determine Iₜₕᵣₑₛₕ for each channel. The difference between the resulting Iₜₕᵣₑₛₕ values and the corresponding baseline are computed by the system 10 and compared against predetermined "safe" and "unsafe" difference values. The Iₜₕᵣₑₛₕ, baseline, and difference values are displayed to the user, along with any other indicia of the safety level determined (such as a red, yellow, green color code), on the display 34, as illustrated in Fig. 28. In MEP Manual mode, the user selects the stimulation current level and the system reports whether or not the stimulation signal evokes a significant response on each channel. Stimulation results may be shown on the display 34 in the form of "YES" and "NO" responses, or other equivalent indicia, as depicted in Fig. 27. Using either mode the surgeon may thus be alerted to potential complications with the spinal cord and any corrective actions deemed necessary may be undertaken at the discretion of the surgeon.

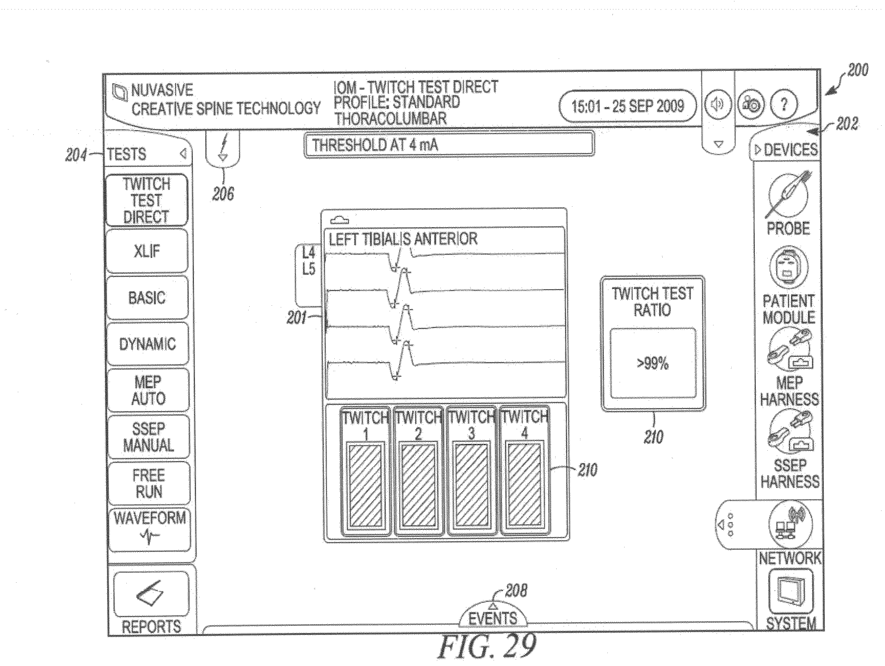

The neuromonitoring system 10 performs neuromuscular pathway (NMP) assessments, via Twitch Test mode, by electrically stimulating a peripheral nerve (preferably the Peroneal Nerve for lumbar and thoracolumbar applications and the Median Nerve for cervical applications) via stimulation electrodes 22 contained in the applicable electrode harness and placed on the skin over the nerve or by direct stimulation of a spinal nerve using a surgical accessory such as the probe 116. Evoked responses from the muscles innervated by the stimulated nerve are detected and recorded, the results of which are analyzed and a relationship between at least two responses or a stimulation signal and a response is identified. The identified relationship provides an indication of the current state of the NMP. The identified relationship may include, but is not necessarily limited to, one or more of magnitude ratios between multiple evoked responses and the presence or absence of an evoked response relative to a given stimulation signal or signals. With reference to Fig. 29, details of the test indicating the state of the NMP and the relative safety of continuing on with nerve testing are conveyed to the surgeon via GUI display 34. On the monitoring screen 200 utilized by the various functions performed by the system 10, function specific data is displayed in a center result area 201. The results may be shown as a numeric value 210, a highlighted label corresponding to the electrode labels 86, or (in the case of twitch test only) a bar graph of the stimulation results. On one side of center result area 201 is a collapsible device menu 202. The device menu displays a graphic representation of each device connected to the patient module 14. Opposite the device menu 202 there is a collapsible test menu 204. The test menu 204 highlights each test that is available under the operable setup profile and may be used to navigate between functions. A collapsible stimulation bar 206 indicates the current stimulation status and provides start and stop stimulation buttons (not shown) to activate and control stimulation. The collapsible event bar 208 stores all the stimulation test results obtained throughout a procedure. Clicking on a particular event will open a note box and annotations may be entered and saved with the response, for later inclusion in a procedure report. The event bar 208 also houses a chat box feature when the system 10 is connected to a remote monitoring system as described above. Within the result area 202 the twitch test specific results may be displayed.

It should be appreciated that while Fig. 29 depicts the monitoring screen 200 while the selected function is the Twitch Test, the features of monitoring screen 200 apply equally to all the functions. Result-specific data is displayed in a center result area 201. A large color saturated numeric value (not shown) is used to show the threshold result. Three different options are provided for showing the stimulation response level. First, the user can view the waveform. Second, a likeness of the color coded electrode harness label 86 may be shown on the display. Third, the color coded label 212 may be integrated with a body image. On one side of center result area 201 there is a collapsible device menu 202. The device menu displays a graphic representation of each device connected to the patient module 14. If a device is selected from the device menu 202, an impedance test may be initiated. Opposite the device menu 202 there is a collapsible test menu 204. The test menu 204 highlights each test that is available under the operable setup profile and may be used to navigate between functions. A collapsible stimulation bar 206 indicates the current stimulation status and provides start and stop stimulation buttons (not shown) to activate and control stimulation. The collapsible event bar 208 stores all the stimulation test results obtained throughout a procedure so that the user may review the entire case history from the monitoring screen. Clicking on a particular event will open a note box and annotations may be entered and saved with the response, for later inclusion in a procedure report chronicling all nerve monitoring functions conducted during the procedure as well as the results of nerve monitoring. In one embodiment the report may be printed immediately from one or more printers located in the operating room or copied to any of a variety of memory devices known in the prior art, such as, by way of example only, a floppy disk, and/or USB memory stick. The system 10 may generate either a full report or a summary report depending on the particular needs of the user. In one embodiment, the identifiers used to identify the surgical accessories to the patient module may also be encoded to identify their lot number or other identifying information. As soon as the accessory is identified, the lot number may be automatically added to the report. Alternatively, hand held scanners can be provided and linked to the control unit 12 or patient module 14. The accessory packaging may be scanned and again the information may go directly to the procedure report. The event bar 208 also houses a chat box feature when the system 10 is connected to a remote monitoring system to allow a user in the operating room to contemporaneously communicate with a person performing the associated neuromonitoring in a remote location.

The neuromonitoring system 10 may test the integrity of pedicle holes (during and/or after formation) and/or screws (during and/or after introduction) via the Basic Stimulation EMG and Dynamic Stimulation EMG tests. To perform the Basic Stimulation EMG a test probe 116 is placed in the screw hole prior to screw insertion or placed on the installed screw head and a stimulation signal is applied. The insulating character of bone will prevent the stimulation current, up to a certain amplitude, from communicating with the nerve, thus resulting in a relatively high Iₜₕᵣₑₛₕ, as determined via the basic threshold hunting algorithm described below. However, in the event the pedicle wall has been breached by the screw or tap, the current density in the breach area will increase to the point that the stimulation current will pass through to the adjacent nerve roots and they will depolarize at a lower stimulation current, thus Iₜₕᵣₑₛₕ will be relatively low. The system described herein may exploit this knowledge to inform the practitioner of the current Iₜₕᵣₑₛₕ of the tested screw to determine if the pilot hole or screw has breached the pedicle wall.

In Dynamic Stim EMG mode, test probe 116 may be replaced with a clip 18 which may be utilized to couple a surgical tool, such as for example, a tap member 28 or a pedicle access needle 26, to the neuromonitoring system 10. In this manner, a stimulation signal may be passed through the surgical tool and pedicle integrity testing can be performed while the tool is in use. Thus, testing may be performed during pilot hole formation by coupling the access needle 26 to the neuromonitoring system 10, and during pilot hole preparation by coupling the tap 28 to the system 10. Likewise, by coupling a pedicle screw to the neuromonitoring system 10 (such as via pedicle screw instrumentation), integrity testing may be performed during screw introduction.

In both Basic Stimulation EMG mode and Dynamic Stimulation EMG mode, the signal characteristics used for testing in the lumbar testing may not be effective when monitoring in the thoracic and/or cervical levels because of the proximity of the spinal cord to thoracic and cervical pedicles. Whereas a breach formed in a pedicle of the lumbar spine results in stimulation being applied to a nerve root, a breach in a thoracic or cervical pedicle may result in stimulation of the spinal cord instead, but the spinal cord may not respond to a stimulation signal the same way the nerve root would. To account for this, the surgical system 10 is equipped to deliver stimulation signals having different characteristics based on the region selected. By way of example only, when the lumbar region is selected, stimulation signals for the stimulated EMG modes comprise single pulse signals. On the other hand, when the thoracic and cervical regions are selected the stimulation signals may be configured as multipulse signals.

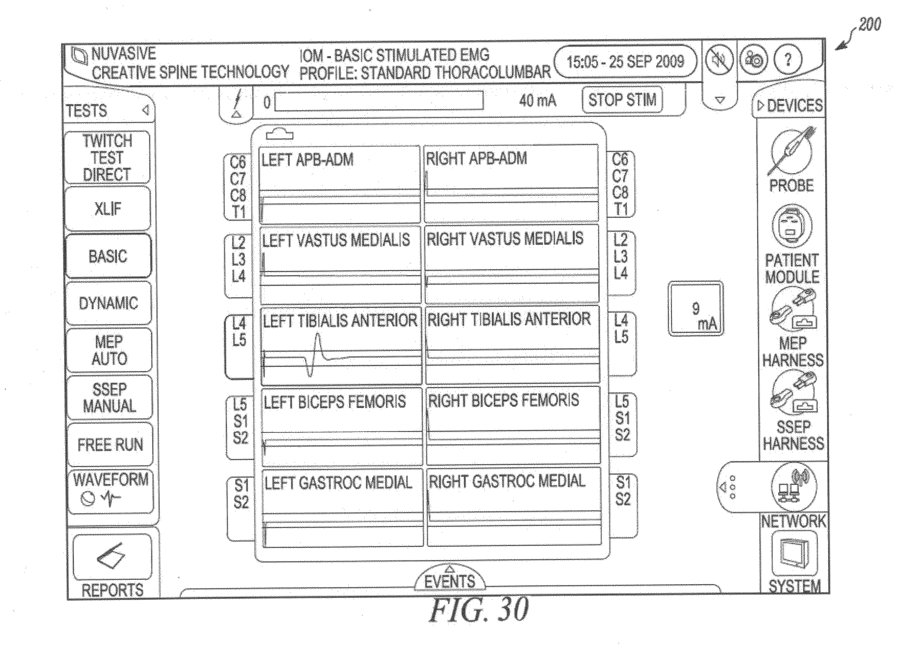

Stimulation results (including but not necessarily limited to at least one of the numerical Iₜₕᵣₑₛₕ value and color coded safety level indication) and other relevant data are conveyed to the user on at least main display 34, as illustrated in Figs. 29 and 30. Fig. 29 illustrates the monitoring screen 200 with the Basic Stimulation EMG test selected. Fig. 30 illustrates the monitoring screen 200 with the Dynamic Stimulation EMG test selected. In one embodiment of the various screw test functions (e.g. Basic and Dynamic), green corresponds to a threshold range of greater than 10 milliamps (mA), a yellow corresponds to a stimulation threshold range of 7-10 mA, and a red corresponds to a stimulation threshold range of 6 mA or below. EMG channel tabs may be selected via the touch screen display 26 to show the Iₜₕᵣₑₛₕ of the corresponding nerves. Additionally, the EMG channel possessing the lowest Iₜₕᵣₑₛₕ may be automatically highlighted and/or colored to clearly indicate this fact to the user.

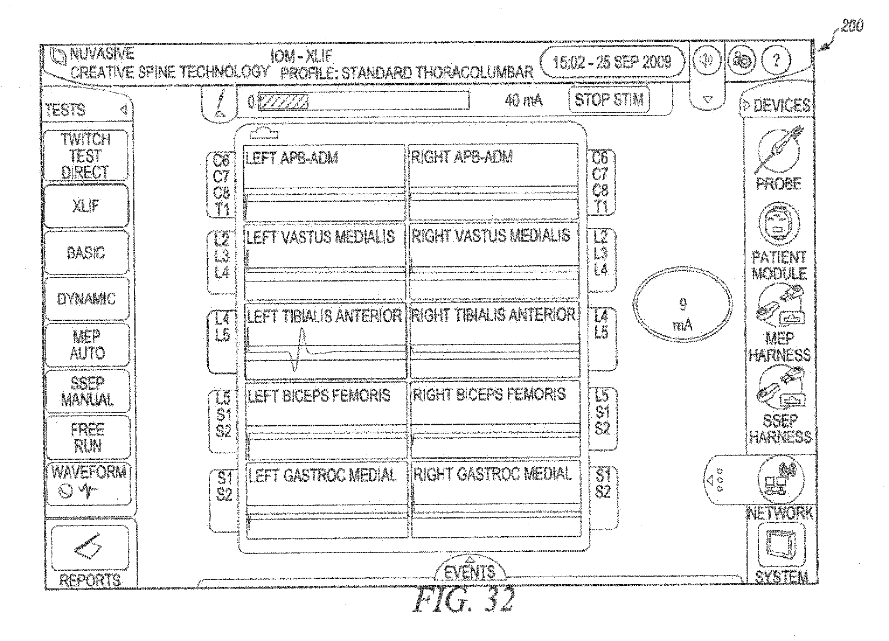

The neuromonitoring system 10 may perform nerve proximity testing, via the XLIF mode, to ensure safe and reproducible access to surgical target sites. Using the surgical access components 26-32, the system 10 detects the existence of neural structures before, during, and after the establishment of an operative corridor through (or near) any of a variety of tissues having such neural structures which, if contacted or impinged, may otherwise result in neural impairment for the patient. The surgical access components 26-32 are designed to bluntly dissect the tissue between the patient's skin and the surgical target site. Dilators of increasing diameter, which are equipped with one or more stimulating electrodes, are advanced towards the target site until a sufficient operating corridor is established to advance retractor 32 to the target site. As the dilators are advanced to the target site electrical stimulation signals are emitted via the stimulation electrodes. The stimulation signal will stimulate nerves in close proximity to the stimulation electrode and the corresponding EMG response is monitored. As a nerve gets closer to the stimulation electrode, the stimulation current required to evoke a muscle response decreases because the resistance caused by human tissue will decrease, and it will take less current to cause nervous tissue to depolarize. Iₜₕᵣₑₛₕ is calculated, using the basic threshold hunting algorithm described below, providing a measure of the communication between the stimulation signal and the nerve and thus giving a relative indication of the proximity between access components and nerves. An example of the monitoring screen 200 with XLIF mode active is depicted in Fig. 32. In a preferred embodiment, a green or safe level corresponds to a stimulation threshold range of 10 milliamps (mA) or greater, a yellow level denotes a stimulation threshold range of 5-9 mA, and a red level denotes a stimulation threshold range of 4 mA or below.

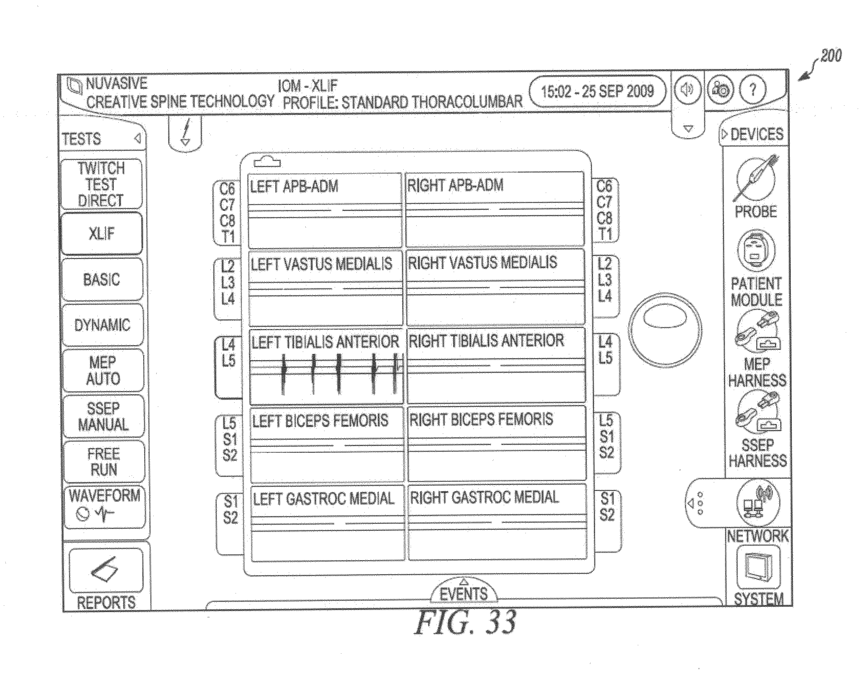

The neuromonitoring system 10 may also conduct free-run EMG monitoring while the system is in any of the above-described modes. Free-run EMG monitoring continuously listens for spontaneous muscle activity that may be indicative of potential danger. The system 10 may automatically cycle into free-run monitoring after 5 seconds (by way of example only) of inactivity. Initiating a stimulation signal in the selected mode will interrupt the free-run monitoring until the system 10 has again been inactive for five seconds, at which time the free-run begins again. An example of the monitoring screen 200 with Free-run EMG active is depicted in Fig. 33.

The neuromonitoring system 10 may also perform a navigated guidance function. The navigated guidance feature may be used by way of example only, to ensure safe and reproducible pedicle screw placement by monitoring the axial trajectory of surgical instruments used during pilot hole formation and/or screw insertion. Preferably, EMG monitoring may be performed simultaneously with the navigated guidance feature. To perform the navigated guidance and angle-measuring device (hereafter "tilt sensor") 54 is connected to the patient module 14 via one of the accessory ports 62. The tilt sensor measures its angular orientation with respect to a reference axis (such as, for example, "vertical" or "gravity") and the control unit displays the measurements. Because the tilt sensor is attached to a surgical instrument the angular orientation of the instrument, may be determined as well, enabling the surgeon to position and maintain the instrument along a desired trajectory during use. In general, to orient and maintain the surgical instrument along a desired trajectory during pilot hole formation, the surgical instrument is advanced to the pedicle (through any of open, mini-open, or percutaneous access) while oriented in the zero-angle position. The instrument is then angulated in the sagittal plane until the proper cranial-caudal angle is reached. Maintaining the proper cranial-caudal angle, the surgical instrument may then be angulated in the transverse plane until the proper medial-lateral angle is attained. Once the control unit 12 indicates that both the medial-lateral and cranial caudal angles are matched correctly, the instrument may be advanced into the pedicle to form the pilot hole, monitoring the angular trajectory of the instrument until the hole formation is complete.

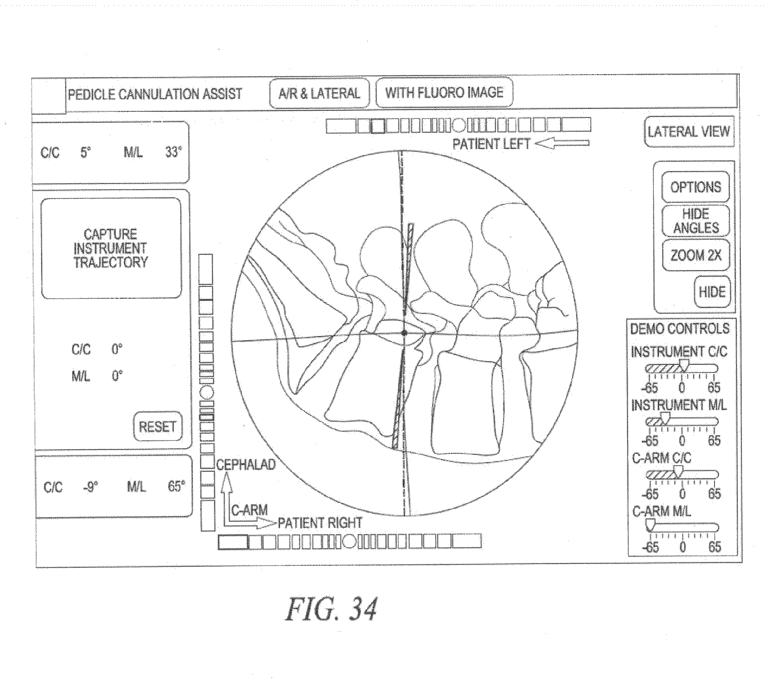

The control unit 12 may communicate any of numerical, graphical, and audio feedback corresponding to the orientation of the tilt sensor in the sagittal plane (cranial-caudal angle) and in the transverse plane (medial-lateral angle). The medial-lateral and cranial-caudal angle readouts may be displayed simultaneously and continuously while the tilt sensor is in use, or any other variation thereof (e.g. individually and/or intermittently). Fig. 34 illustrates, by way of example only, one embodiment of a GUI screen for the Navigated Guidance function. The angular orientation of the instrument is displayed along with a color coded targeting scheme to help the user find the desired angle.

To obtain Iₜₕᵣₑₛₕ and take advantage of the useful information it provides, the system 10 identifies and measures the peak-to-peak voltage (Vₚₚ) of each EMG response corresponding to a given stimulation current (I_{Stim}). Identifying the true Vₚₚ of a response may be complicated by the existence of stimulation and/or noise artifacts which may create an erroneous Vₚₚ measurement. To overcome this challenge, the neuromonitoring system 10 of the present invention may employ any number of suitable artifact rejection techniques such as those shown and described in full in the above referenced co-pending and commonly assigned PCT App. Ser. No. PCT/US2004/025550, entitled "System and Methods for Performing Dynamic Pedicle Integrity Assessments," filed on August 5, 2004.

Upon measuring Vₚₚ for each EMG response, the Vₚₚ information is analyzed relative to the corresponding stimulation current (Iₛₜᵢₘ) in order to identify the minimum stimulation current (I_{Thresh}) capable of resulting in a predetermined Vₚₚ EMG response. According to the present invention, the determination of I_{Thresh} may be accomplished via any of a variety of suitable algorithms or techniques.

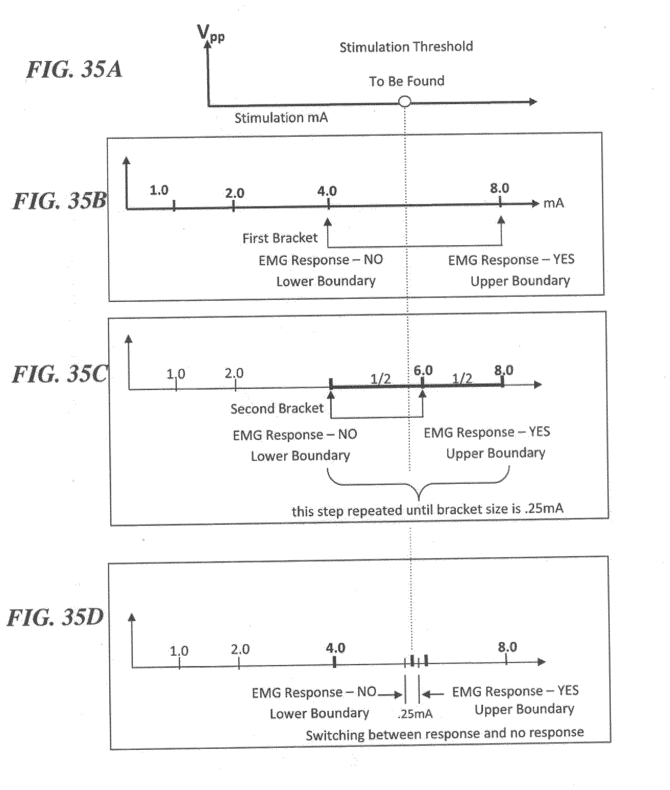

Figs. 35 A-D illustrate, by way of example only, the principles of a threshold hunting algorithm of the present invention used to quickly find I_{Thresh}. The method for finding I_{Thresh} utilizes a bracketing method and a bisection method. The bracketing method quickly finds a range (bracket) of stimulation currents that must contain I_{Thresh} and the bisection method narrows the bracket until I_{Thresh} is known within a specified accuracy. If the stimulation current threshold, I_{Thresh}, of a channel exceeds a maximum stimulation current, that threshold is considered out of range.

Figs. 35 A-D illustrate the bracketing feature of the threshold hunting algorithm of the present invention. Stimulation begins at a minimum stimulation current, such as (by way of example only) 1mA. It will be appreciated that the relevant current values depend in part on the function performed (e.g. high currents are used for MEP and low currents are generally used for other functions) and the current values described here are for purposes of example only and may in actuality be adjusted to any scale. The level of each subsequent stimulation is doubled from the preceding stimulation level until a stimulation current recruits (i.e. results in an EMG response with a Vₚₚ greater or equal to Vₜₕᵣₑₛₕ). The first stimulation current to recruit (8 mA in Fig. 35 B), together with the last stimulation current to have not recruited (4 mA in Fig. 35 B), forms the initial bracket.

Figs. 35 C-D illustrate the bisection feature of the threshold hunting algorithm of the present invention. After the threshold current I_{Thresh} has been bracketed (Fig. 35 B), the initial bracket is successively reduced via bisection to a predetermined width, such as (by way of example only) 0.25 mA. This is accomplished by applying a first bisection stimulation current that bisects (i.e. forms the midpoint of) the initial bracket (6 mA in Fig. 35 C). If this first bisection stimulation current recruits, the bracket is reduced to the lower half of the initial bracket (e.g. 4 mA and 6 mA in Fig. 35C). If this first bisection stimulation current does not recruit, the bracket is reduced to the upper half of the initial bracket (e.g. 6 mA and 8 mA in Fig. 35 C). This process is continued for each successive bracket until I_{Thresh} is bracketed by stimulation currents separated by the predetermined width (which, in this case, is 0.25 mA). In this example shown, this would be accomplished by applying a second bisection stimulation current (forming the midpoint of the second bracket, or 5 mA in this example). Because this second bisection stimulation current is below I_{Thresh}, it will not recruit. As such, the second bracket will be reduced to the upper half thereof (5 mA to 6 mA), forming a third bracket. A third bisection stimulation current forming the mid-point of the third bracket (5.50 mA in this case) will then be applied. Because this third bisection stimulation current is below I_{Thresh}, it will not recruit. As such, the third bracket will be reduced to the upper half thereof (5.50 mA to 6 mA), forming a fourth bracket. A fourth bisection stimulation current forming the mid-point of the fourth bracket (5.75 mA in this case) will then be applied. Because the fourth bisection stimulation current is above I_{Thresh}, it will recruit. The final bracket is therefore between 5.50mA and 5.75 mA. Due to the "response" or recruitment at 5.50 mA and "no response" or lack of recruitment at 5.75 mA, it can be inferred that I_{Thresh} is within this range. In one embodiment, the midpoint of this final bracket may be defined as I_{Thresh}, however, any value falling within the final bracket may be selected as I_{Thresh} without departing from the scope of the present invention. Depending on the active mode, the algorithm may stop after finding I_{Thresh} for the first responding channel (i.e. the channel with the lowest I thresh) or the bracketing and bisection steps may be repeated for each channel to determine I_{Thresh} for each channel. In one embodiment, this multiple channel I_{Thresh} determination may be accomplished by employing the additional steps of the multi-channel threshold detection algorithm, described below.

Additionally, in the "dynamic" functional modes, including, but not necessarily limited to Dynamic Stimulation EMG and XLIF, the system may continuously update the stimulation threshold level and indicate that level to the user. To do so, the threshold hunting algorithm does not repeatedly determine the I_{Thresh} level anew, but rather, it determines whether stimulation current thresholds are changing. This is accomplished, as illustrated in Fig. 35 D, by a monitoring phase that involves switching between stimulations at lower and upper ends of the final bracket. If the threshold has not changed then the lower stimulation current should not evoke a response, while the upper end of the bracket should. If either of these conditions fail, the bracket is adjusted accordingly. The process is repeated for each of the active channels to continue to assure that each threshold is bracketed. If stimulations fail to evoke the expected response three times in a row, then the algorithm transitions back to the bracketing state in order to reestablish the bracket. In the event a change in I_{Thresh} is detected during the monitoring phase, the user may be alerted immediately via the screen display and/or audio feedback. By way of example only, the color shown on the display corresponding to the previous I_{Thresh} can be altered to a neutral color (e.g. black, grey, etc...) as soon as the change in I_{Thresh} is detected but before the new I_{Thresh} value is determined. If an audio tone is used to represent a particular safety level, the tone can ceased as soon as the change in detected. Once the new I_{Thresh} value is determined the color and/or audio tone can be altered again to signify the value.

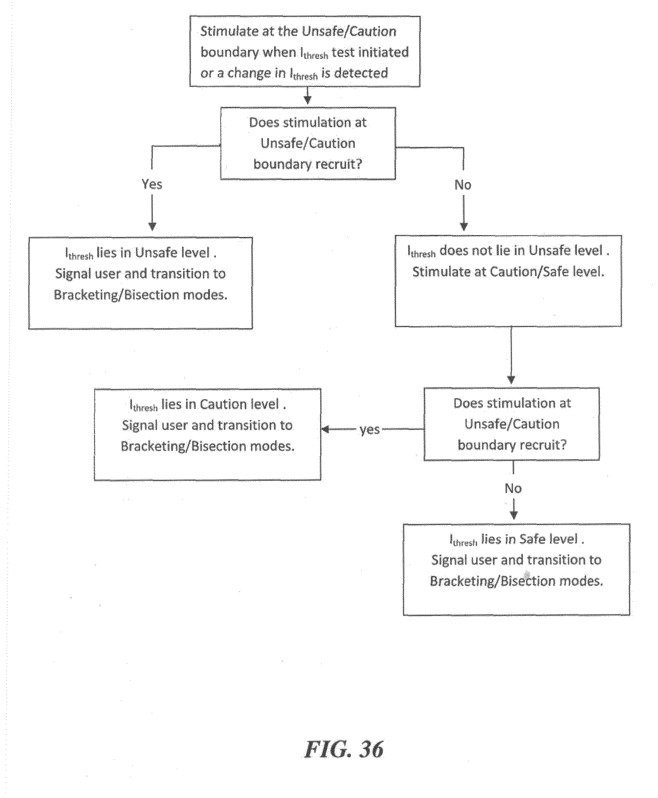

In an alternative embodiment, rather than beginning by entering the bracketing phase at the minimum stimulation current and bracketing upwards until I_{Thresh} is bracketed, the threshold hunting algorithm may begin by immediately determining the appropriate safety level and then entering the bracketing phase. The algorithm may accomplish this by initiating stimulation at one or more of the boundary current levels. By way of example only, and with reference to Fig. 36, the algorithm may begin by delivering a stimulation signal at the boundary between the unsafe (e.g. red) and caution (e.g. yellow) levels. If the safety level is not apparent after the first stimulation, the algorithm may stimulate again at the boundary between the caution (e.g. yellow) and safe (e.g. green) levels. Once the safety level is known (i.e. after the first stimulation if the safety level is red, or, after the second stimulation if the safety level is yellow or green) the screen display may be updated to the appropriate color and/or coded audio signals may be emitted. As the screen display is updated, the algorithm may transition to the bracketing and bisection phases to determine the actual I_{Thresh} value. When the I_{Thresh} value is determined the display may be updated again to reflect the additional information. In dynamic modes, if the monitoring phase detects a change in I_{Thresh}, the algorithm will again stimulate at the boundary level(s) as necessary and update the color and/or audio signals before transitioning to the bracketing and bisection phases to determine the new I_{Thresh}.

For some functions, such as (by way of example) MEP, it may be desirable to obtain I_{Thresh} for each active channel each time the function is performed. This is particularly advantageous when assessing changes in I_{Thresh} over time as a means to detect potential problems (as opposed to detecting an I_{Thresh} below a predetermined level determined to be safe, such as in the Stimulated EMG modes). While I_{Thresh} can be found for each active channel using the algorithm as described above, it requires a potentially large number of stimulations, each of which is associated with a specific time delay, which can add significantly to the response time. Done repeatedly, it could also add significantly to the overall time required to complete the surgical procedure, which may present added risk to the patient and added costs. To overcome this drawback, a preferred embodiment of the neuromonitoring system 10 boasts a multi-channel threshold hunting algorithm so as to quickly determine I_{Thresh} for each channel while minimizing the number of stimulations and thus reduce the time required to perform such determinations.

The multi-channel threshold hunting algorithm reduces the number stimulations required to complete the bracketing and bisection steps when I_{Thresh} is being found for multiple channels. The multi-channel algorithm does so by omitting stimulations for which the result is predictable from the data already acquired. When a stimulation signal is omitted, the algorithm proceeds as if the stimulation had taken place. However, instead of reporting an actual recruitment result, the reported result is inferred from previous data. This permits the algorithm to proceed to the next step immediately, without the time delay associated with a stimulation signal.

Regardless of what channel is being processed for I_{Thresh}, each stimulation signal elicits a response from all active channels. That is to say, every channel either recruits or does not recruit in response to a stimulation signal (again, a channel is said to have recruited if a stimulation signal evokes an EMG response deemed to be significant on that channel, such as Vₚₚ of approximately 100 uV). These recruitment results are recorded and saved for each channel. Later, when a different channel is processed for I_{Thresh}, the saved data can be accessed and, based on that data, the algorithm may omit a stimulation signal and infer whether or not the channel would recruit at the given stimulation current.

There are two reasons the algorithm may omit a stimulation signal and report previous recruitment results. A stimulation signal may be omitted if the selected stimulation current would be a repeat of a previous stimulation. By way of example only, if a stimulation current of 1mA was applied to determine I_{Thresh} for one channel, and a stimulation at 1mA is later required to determine I_{Thresh} for another channel, the algorithm may omit the stimulation and report the previous results. If the specific stimulation current required has not previously been used, a stimulation signal may still be omitted if the results are already clear from the previous data. By way of example only, if a stimulation current of 2mA was applied to determine I_{Thresh} for a previous channel and the present channel did not recruit, when a stimulation at 1 mA is later required to determine I_{Thresh} for the present channel, the algorithm may infer from the previous stimulation that the present channel will not recruit at 1mA because it did not recruit at 2mA. The algorithm may therefore omit the stimulation and report the previous result.

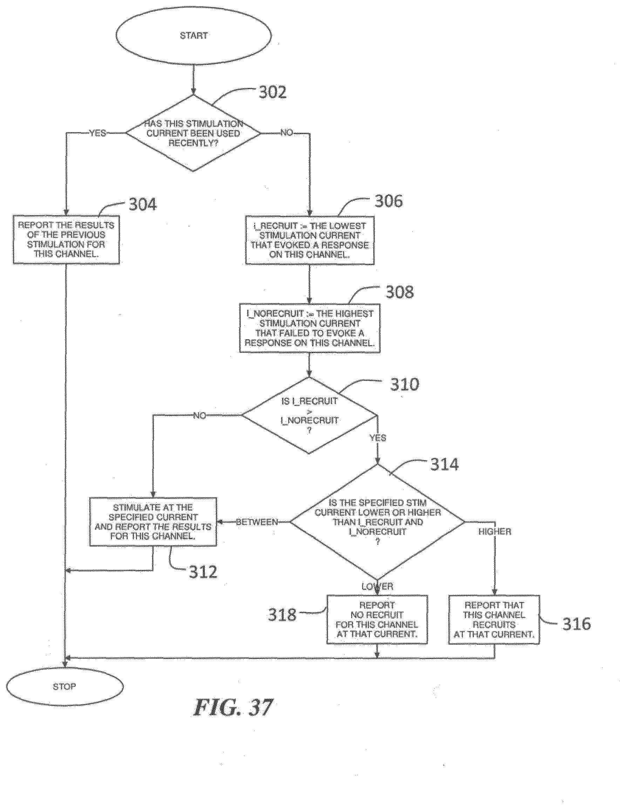

Fig. 37 illustrates (in flowchart form) a method by which the multi-channel threshold hunting algorithm determines whether to stimulate, or not stimulate and simply report previous results. The algorithm first determines if the selected stimulation current has already been used (step 302). If the stimulation current has been used, the stimulation is omitted and the results of the previous stimulation are reported for the present channel (step 304). If the stimulation current has not been used, the algorithm determines Irecruit (step 306) and I_{norecruit} (step 308) for the present channel. Irecruit is the lowest stimulation current that has recruited on the present channel. I_{norecruit} is the highest stimulation current that has failed to recruit on the present channel. The algorithm next determines whether Irecruit is greater than I_{norecruit} (step 310). An Irecruit that is not greater than I_{norecruit} is an indication that changes have occurred to I_{Thresh} on that channel. Thus, previous results may not be reflective of the present threshold state and the algorithm will not use them to infer the response to a given stimulation current. The algorithm will stimulate at the selected current and report the results for the present channel (step 312). If Irecruit is greater than I_{norecruit}, the algorithm determines whether the selected stimulation current is higher than Irecruit, lower than I_{norecruit}, or between Irecruit and I_{norecruit} (step 314). If the selected stimulation current is higher than Irecruit, the algorithm omits the stimulation and reports that the present channel recruits at the specified current (step 316). If the selected stimulation current is lower than I_{norecruit}, the algorithm infers that the present channel will not recruit at the selected current and reports that result (step 318). If the selected stimulation current falls between Irecruit and I_{norecruit}, the result of the stimulation cannot be inferred and the algorithm stimulates at the selected current and reports the results for the present channel (step 312). This method may be repeated until I_{Thresh} has been determined for every active channel.

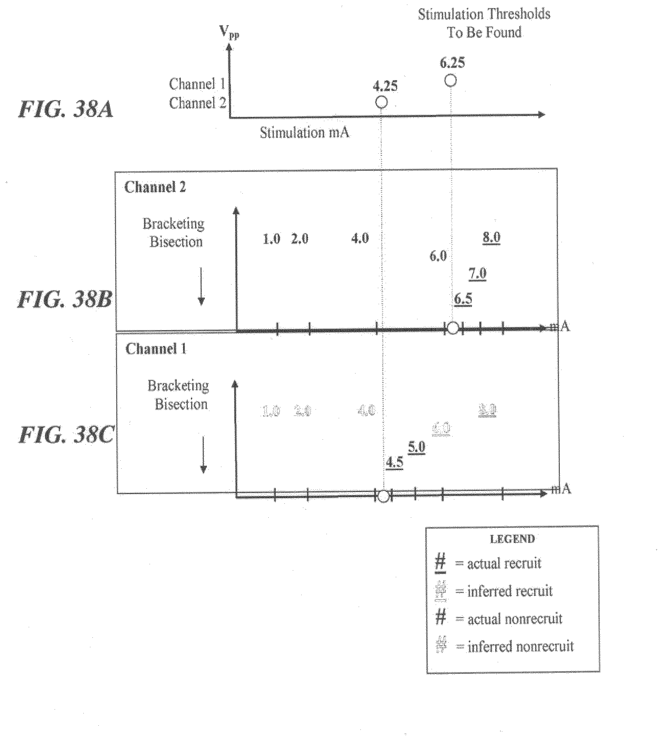

In the interest of clarity, Figs. 38 A-C demonstrate use of the multi-channel threshold hunting algorithm to determine Iₜₕᵣₑₛₕ on only two channels. It should be appreciated, however, that the multi-channel algorithm is not limited to finding Iₜₕᵣₑₛₕ for two channels, but rather it may be used to find Iₜₕᵣₑₛₕ for any number of channels, such as (for example) eight channels according to a preferred embodiment of the neuromonitoring system 10. With reference to Fig. 38 A, channel 1 has an Iₜₕᵣₑₛₕ to be found of 6.25 mA and channel 2 has an Iₜₕᵣₑₛₕ to be found of 4.25 mA. Iₜₕᵣₑₛₕ for channel 1 is found first as illustrated in Fig. 38 B, using the bracketing and bisection methods discussed above. Bracketing begins at the minimum stimulation current (for the purposes of example only) of 1 mA. As this is the first channel processed and no previous recruitment results exist, no stimulations are omitted. The stimulation current is doubled with each successive stimulation until a significant EMG response is evoked at 8 mA. The initial bracket of 4-8 mA is bisected, using the bisection method described above, until the stimulation threshold, Iₜₕᵣₑₛₕ, is contained within a final bracket separated by the selected width or resolution (again .25 mA). In this example, the final bracket is 6 mA-6.25 mA. Iₜₕᵣₑₛₕ may be defined as any point within the final bracket or as the midpoint of the final bracket (6.125 mA in this case). In either event, Iₜₕᵣₑₛₕ is selected and reported as Iₜₕᵣₑₛₕ for channel 1.

Once Iₜₕᵣₑₛₕ is found for channel 1, the algorithm turns to channel 2, as illustrated in Fig. 38 C. The algorithm begins to process channel 2 by determining the initial bracket, which is again 4-8 mA. All the stimulation currents required in the bracketing state were used in determining Iₜₕᵣₑₛₕ for channel 1. The algorithm refers back to the saved data to determine how channel 1 responded to the previous stimulations. From the saved data, the algorithm may infer that channel 2 will not recruit at stimulation currents of 1, 2, and 4 mA, and will recruit at 8 mA. These stimulations are omitted and the inferred results are displayed. The first bisection stimulation current selected in the bisection process (6 mA in this case), was previously used and, as such, the algorithm may omit the stimulation and report that channel 2 recruits at that stimulation current. The next bisection stimulation current selected (5 mA in this case) has not been previously used and, as such, the algorithm must determine whether the result of a stimulation at 5 mA may still be inferred. In the example shown, I_{recruit} and I_{norecruit} t are determined to be 6 mA and 4 mA, respectively. Because 5 mA falls in between Irecruit and I_{norecruit}, the algorithm may not infer the result from the previous data and, as such, the stimulation may not be omitted. The algorithm then stimulates at 5 mA and reports that the channel recruits. The bracket is reduced to the lower half (making 4.50 mA the next bisection stimulation current). A stimulation current of 4.5 mA has not previously been used and, as such, the algorithm again determines Irecruit and I_{norecruit} (5 mA and 4 mA in this case). The selected stimulation current (4.5 mA) falls in between Irecruit an I_{norecruit} and, as such, the algorithm stimulates at 4.5 mA and reports the results. The bracket now stands at its final width of .25 mA (for the purposes of example only). Iₜₕᵣₑₛₕ may be defined as any point within the final bracket or as the midpoint of the final bracket (4.125 mA in this case). In either event, Iₜₕᵣₑₛₕ is selected and reported as Iₜₕᵣₑₛₕ for channel 2.

Although the multi-channel threshold hunting algorithm is described above as processing channels in numerical order, it will be understood that the actual order in which channels are processed is immaterial. The channel processing order may be biased to yield the highest or lowest threshold first (discussed below) or an arbitrary processing order may be used. Furthermore, it will be understood that it is not necessary to complete the algorithm for one channel before beginning to process the next channel, provided that the intermediate state of the algorithm is retained for each channel. Channels are still processed one at a time. However, the algorithm may cycle between one or more channels, processing as few as one stimulation current for that channel before moving on to the next channel. By way of example only, the algorithm may stimulate at 10mA while processing a first channel for Iₜₕᵣₑₛₕ. Before stimulating at 20 mA (the next stimulation current in the bracketing phase), the algorithm may cycle to any other channel and process it for the 10 mA stimulation current (omitting the stimulation if applicable). Any or all of the channels may be processed this way before returning to the first channel to apply the next stimulation. Likewise, the algorithm need not return to the first channel to stimulate at 20 mA, but instead may select a different channel to process first at the 20 mA level. In this manner, the algorithm may advance all channels essentially together and bias the order to find the lower threshold channels first or the higher threshold channels first. By way of example only, the algorithm may stimulate at one current level and process each channel in turn at that level before advancing to the next stimulation current level. The algorithm may continue in this pattern until the channel with the lowest Iₜₕᵣₑₛₕ is bracketed. The algorithm may then process that channel exclusively until Iₜₕᵣₑₛₕ is determined, and then return to processing the other channels one stimulation current level at a time until the channel with the next lowest Iₜₕᵣₑₛₕ is bracketed. This process may be repeated until Iₜₕᵣₑₛₕ is determined for each channel in order of lowest to highest Iₜₕᵣₑₛₕ. If Iₜₕᵣₑₛₕ for more than one channel falls within the same bracket, the bracket may be bisected, processing each channel within that bracket in turn until it becomes clear which one has the lowest Iₜₕᵣₑₛₕ. If it becomes more advantageous to determine the highest Iₜₕᵣₑₛₕ first, the algorithm may continue in the bracketing state until the bracket is found for every channel and then bisect each channel in descending order.

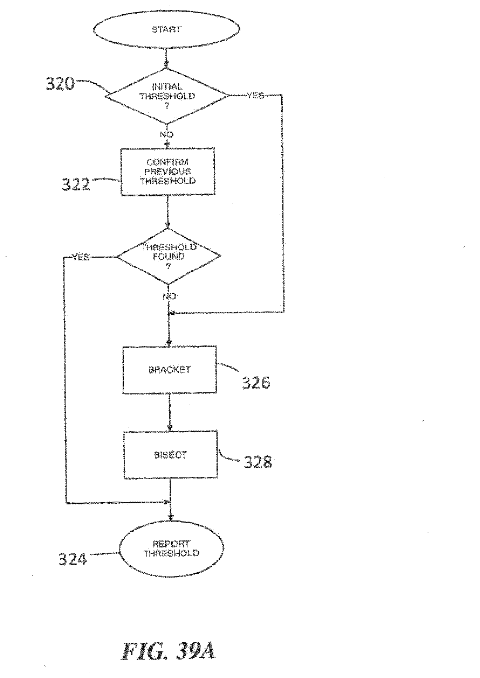

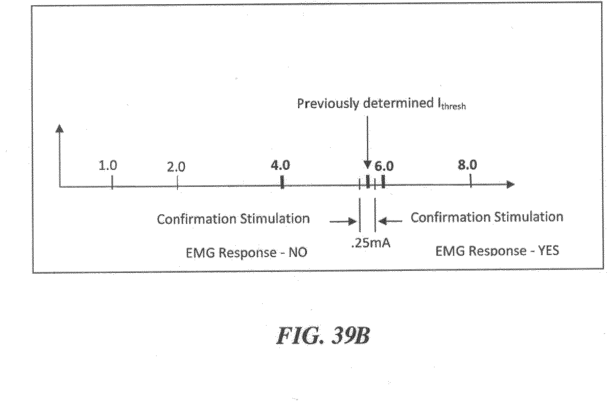

Figs. 39A-B illustrates a further feature of the threshold hunting algorithm of the present invention, which advantageously provides the ability to further reduce the number of stimulations required to find Iₜₕᵣₑₛₕ when an Iₜₕᵣₑₛₕ value has previously been determined for a specific channel. In the event that a previous Iₜₕᵣₑₛₕ determination exists for a specific channel, the algorithm may begin by merely confirming the previous Iₜₕᵣₑₛₕ rather than beginning anew with the bracketing and bisection methods. The algorithm first determines whether it is conducting the initial threshold determination for the channel or whether there is a previous Iₜₕᵣₑₛₕ determination (step 320). If it is not the initial determination, the algorithm confirms the previous determination (step 322) as described below. If the previous threshold is confirmed, the algorithm reports that value as the present Iₜₕᵣₑₛₕ (step 324). If it is the initial Iₜₕᵣₑₛₕ determination, or if the previous threshold cannot be confirmed, then the algorithm performs the bracketing function (step 326) and bisection function (step 328) to determine Iₜₕᵣₑₛₕ and then reports the value (step 324).

Although the hunting algorithm is discussed herein in terms of finding Iₜₕᵣₑₛₕ (the lowest stimulation current that evokes a predetermined EMG response), it is contemplated that alternative stimulation thresholds may be useful in assessing the health of the spinal cord or nerve monitoring functions and may be determined by the hunting algorithm. By way of example only, the hunting algorithm may be employed by the system 10 to determine a stimulation voltage threshold, Vₛₜᵢₘₜₕᵣₑₛₕ. This is the lowest stimulation voltage (as opposed to the lowest stimulation current) necessary to evoke a significant EMG response, Vₜₕᵣₑₛₕ. Bracketing, bisection and monitoring states are conducted as described above for each active channel, with brackets based on voltage being substituted for the current based brackets previously described. Moreover, although described above within the context of MEP monitoring, it will be appreciated that the algorithms described herein may also be used for determining the stimulation threshold (current or voltage) for any other EMG related functions, including but not limited to pedicle integrity (screw test), nerve detection, and nerve root retraction.

The present invention may be implemented using any combination of computer programming software, firmware or hardware. As a preparatory step to practicing the invention or constructing an apparatus according to the invention, the computer programming code (whether software or firmware) according to the invention will typically be stored in one or more machine readable storage mediums such as fixed (hard) drives, diskettes, optical disks, magnetic tape, semiconductor memories such as ROMs, PROMs, etc., thereby making an article of manufacture in accordance with the invention. The article of manufacture containing the computer programming code is used by either executing the code directly from the storage device, by copying the code from the storage device into another storage device such as a hard disk, RAM, etc. or by transmitting the code on a network for remote execution. As can be envisioned by one of skill in the art, many different combinations of the above may be used. Accordingly the present invention is limited by the scope of the appended claims.

## Claims

1. A system (10) for performing somatosensory evoked potential monitoring during surgery, comprising:
a stimulator (22) configured to deliver an electrical stimulation signal to a peripheral nerve of a patient;
at least one sensor (24) configured to detect somatosensory responses evoked by said electrical stimulation signal;
a control unit (12) in communication with said stimulator and said sensor, said control unit being configured to
(a) direct transmission of the stimulation signal,
(b) receive the evoked somatosensory response data from the sensor,
(c) automatically optimize signal parameters of the stimulation signal based on the evoked somatosensory response data;
(d) assess spinal cord health by identifying a relationship between the somatosensory response to a first stimulation signal and a subsequent somatosensory response to a second stimulation signal wherein the relationship being at least one of a change in latency and a change in amplitude between the first somatosensory response and the subsequent somatosensory response, and
(e) communicate the assessment to the user;
**characterised in that**
(f) the control unit is configured to direct transmission of a stimulation signal to at least 4 different stimulation sites each located on a different one of the patient's limbs and identify the relationship for each stimulation site; and
(g) the control unit is configured to deliver initial stimulation to each of the 4 different stimulation sites each located on a different one of the patient's limbs and set an initial SSEP stimulus intensity based on the responses to the initial stimulation,
(h) wherein the neuromonitoring system (10) is configured to stimulate peripheral sensory nerves that exit the spinal cord below the level of surgery and then to measure the electrical action potential from electrodes located on the nervous system superior to the surgical target site.

2. The system of Claim 1, wherein the assessment is further communicated by displaying a color associated with the assessment.

3. The system of Claim 2, wherein the color displayed is one of Green, Yellow, and Red.

4. The system according to any of Claims 1-3, wherein the relationship identified is both a change in latency and a change in amplitude between the first somatosensory response and the subsequent somatosensory response.

5. The system according to any of Claims 1-4, wherein the control unit (12) is configured to receive instructions from a user to optimize at least one parameter associated with the stimulation signal.

6. The system of Claim 5, wherein the at least one of the pulse number, pulse width, pulse rate, and pulse current level may be optimized.

7. The system of Claim 1, wherein the control unit (12) performs a threshold hunting algorithm to optimize the stimulation signal.

8. The system of Claim 7, wherein the threshold hunting algorithm is based on successive approximation.

9. The system of Claim 8, wherein the successive approximation involves:
(a) establishing a bracket within which the lowest stimulation current is contained; and
(b) successively bisecting the bracket until the lowest stimulation current is determined within a specified accuracy.

10. The system according to any of Claims 1-9, further comprising a display in communication with the control unit (12) for visually communicating to said user.

11. The system of Claim 10, wherein the display includes touch-screen control capabilities to allow the user to interface with the control unit (12).

12. The system according to any of Claims 1-11, wherein the control unit (12) is further configured to perform at least one of stimulated EMG and MEP assessments.

13. The system according to any of Claims 1-12, wherein the assessment is communicated by displaying at least one possible cause for a change in relationship between the somatosensory response to said first stimulation signal and the subsequent somatosensory response to said second stimulation signal.

## Patentansprüche

1. System (10) zur Durchführung einer somatosensorisch evozierten Potenzial-überwachung während einer Operation, umfassend:
- einen Stimulator (22), der eingerichtet ist, um ein elektrisches Stimulationssignal an einen peripheren Nerv eines Patienten abzugeben;
- zumindest einen Sensor (24), der eingerichtet ist, um somatosensorische Reaktionen, die durch das elektrische Stimulationssignal hervorgerufen werden, zu erfassen,
- eine Steuereinheit (12), die mit dem Stimulator und dem Sensor in Verbindung steht, wobei die Steuereinheit eingerichtet ist, um
- (a) eine Übertragung des Stimulationssignals zu leiten,
- (b) die evozierten somatosensorischen Reaktionsdaten vom Sensor zu empfangen,
- (c) die Signalparameter des Stimulationssignals auf der Grundlage der evozierten somatosensorischen Reaktionsdaten automatisch zu optimieren;
- (d) die Rückenmarksgesundheit durch Identifizieren einer Beziehung zwischen der somatosensorischen Reaktion auf ein erstes Stimulationssignal und einer nachfolgenden somatosensorischen Reaktion auf ein zweites Stimulationssignal zu bewerten, wobei die Beziehung zumindest eine Änderung der Latenz oder eine Änderung der Amplitude zwischen der ersten somatosensorischen Reaktion und der nachfolgenden somatosensorischen Reaktion ist, und
- (e) die Bewertung dem Benutzer mitzuteilen;
- **dadurch gekennzeichnet, dass**
- (f) die Steuereinheit eingerichtet ist, um eine Übertragung eines Stimulationssignals an zumindest vier verschiedenen Stimulationsstellen zu leiten, die jeweils auf einer anderen Gliedmaße des Patienten angeordnet sind, und um die Beziehung für jede Stimulationsstelle zu identifizieren; und
- (g) die Steuereinheit eingerichtet ist, um eine anfängliche Stimulation an jede der vier verschiedenen Stimulationsstellen abzugeben, die jeweils auf einer anderen Gliedmaße des Patienten angeordnet sind, und um eine anfängliche SSEP-Stimulusintensität auf der Grundlage der Reaktionen auf die anfängliche Stimulation festzulegen,
- (h) wobei das Neuromonitoring-System (10) eingerichtet ist, um periphere sensorischen Nerven zu stimulieren, die das Rückenmark unterhalb des Operationsniveaus verlassen, und dann das elektrische Aktionspotential von Elektroden zu messen, die auf dem Nervensystem oberhalb des chirurgischen Zielbereichs angeordnet sind.

2. System nach Anspruch 1, wobei die Bewertung ferner durch Anzeigen einer mit der Bewertung verbundenen Farbe mitgeteilt wird.

3. System nach Anspruch 2, wobei die angezeigte Farbe Grün, Gelb oder Rot ist.

4. System nach einem der Ansprüche 1 bis 3, wobei die identifizierte Beziehung sowohl eine Änderung der Latenz als auch eine Änderung der Amplitude zwischen der ersten somatosensorischen Reaktion und der nachfolgenden somatosensorischen Reaktion ist.

5. System nach einem der Ansprüche 1 bis 4, wobei die Steuereinheit (12) eingerichtet ist, um Anweisungen von einem Benutzer zu empfangen, um zumindest einen mit dem Stimulationssignal verbundenen Parameter zu optimieren.

6. System nach Anspruch 5, wobei zumindest einer der Parameter Impulszahl, Impulsbreite, Impulsrate oder Impulsstrompegel optimiert werden kann.

7. System nach Anspruch 1, wobei die Steuereinheit (12) einen Algorithmus zur Schwellenwertsuche durchführt, um das Stimulationssignal zu optimieren.

8. System nach Anspruch 7, wobei der Algorithmus zur Schwellenwertsuche auf einer sukzessiven Approximation beruht.

9. System nach Anspruch 8, wobei die sukzessive Approximation umfasst:
- (a) Festlegen einer Klammer, in der der niedrigste Stimulationsstrom enthalten ist; und
- (b) sukzessives Halbieren der Klammer, bis der niedrigste Stimulationsstrom innerhalb einer festgelegten Genauigkeit bestimmt ist.

10. System nach einem der Ansprüche 1 bis 9, das ferner eine Anzeige in Kommunikation mit der Steuereinheit (12) zur visuellen Kommunikation mit dem Benutzer aufweist.

11. System nach Anspruch 10, wobei die Anzeige Touchscreen-Bedienungsmöglichkeiten umfasst, um dem Benutzer eine Schnittstelle mit der Steuereinheit (12) zu ermöglichen.

12. System nach einem der Ansprüche 1 bis 11, wobei die Steuereinheit (12) ferner eingerichtet ist, um zumindest eine stimulierte EMG- oder MEP-Bewertung durchzuführen.

13. System nach einem der Ansprüche 1 bis 12, wobei die Bewertung durch Anzeigen zumindest einer möglichen Ursache für eine Änderung der Beziehung zwischen der somatosensorischen Reaktion auf das erste Stimulationssignal und der nachfolgenden somatosensorischen Reaktion auf das zweite Stimulationssignal mitgeteilt wird.

## Revendications

1. Système (10) de surveillance de potentiel évoqué somatosensoriel au cours d'une intervention chirurgicale, comprenant :
un stimulateur (22) configuré pour délivrer un signal de stimulation électrique à un nerf périphérique d'un patient ;
au moins un capteur (24) configuré pour détecter les réponses somatosensorielles évoquées par ledit signal de stimulation électrique ;
une unité de commande (12) en communication avec ledit stimulateur et ledit capteur, ladite unité de commande étant configurée pour
(a) transmettre directement un signal de stimulation,
(b) recevoir les données de réponse somatosensorielle évoquée du capteur,
(c) optimiser automatiquement les paramètres de signal de la stimulation sur la base des données relatives à la réponse somatosensorielle évoquée ;
(d) évaluer la santé de la moelle épinière en identifiant une relation entre la réponse somatosensorielle à un premier signal de stimulation et une réponse somatosensorielle ultérieure à un second signal de stimulation, la relation étant au moins l'une des suivantes : un changement de latence et un changement d'amplitude entre la première réponse somatosensorielle et la réponse somatosensorielle ultérieure, et
(e) communiquer l'évaluation à l'utilisateur ; **caractérisé en ce que**
(f) l'unité de commande est configurée pour diriger la transmission d'un signal de stimulation vers au moins 4 sites de stimulation différents situés chacun sur un membre différent du patient et pour identifier la relation pour chaque site de stimulation ; et
(g) l'unité de commande est configurée pour délivrer une stimulation initiale à chacun des 4 sites de stimulation différents, chacun étant situé sur un membre différent du patient, et pour définir une intensité de stimulation SSEP initiale sur la base des réponses à la stimulation initiale,
(h) le système de neuromonitoring (10) est configuré pour stimuler les nerfs sensoriels périphériques qui sortent de la moelle épinière au-dessous du niveau de l'intervention chirurgicale, puis pour mesurer le potentiel d'action électrique à partir d'électrodes situées sur le système nerveux supérieur au site cible de l'intervention chirurgicale.

2. Système selon la revendication 1, dans lequel l'évaluation est en outre communiquée par l'affichage d'une couleur associée à l'évaluation.

3. Système selon la revendication 2, dans lequel la couleur affichée est l'une des couleurs suivantes : vert, jaune et rouge.

4. Système selon l'une quelconque des revendications 1 à 3, dans lequel la relation identifiée est à la fois un changement de latence et un changement d'amplitude entre la première réponse somatosensorielle et la réponse somatosensorielle suivante.

5. Système selon l'une quelconque des revendications 1 à 4, dans lequel l'unité de commande (12) est configurée pour recevoir des instructions d'un utilisateur afin d'optimiser au moins un paramètre associé au signal de stimulation.

6. Système selon la revendication 5, dans lequel l'au moins un des éléments suivants peut être optimisé : nombre d'impulsions, largeur d'impulsion, fréquence d'impulsion et niveau de courant d'impulsion.

7. Système selon la revendication 1, dans lequel l'unité de commande (12) exécute un algorithme de recherche de seuil pour optimiser le signal de stimulation.

8. Système selon la revendication 7, dans lequel l'algorithme de recherche de seuil est basé sur l'approximation successive.

9. Système selon la revendication 8, dans lequel l'approximation successive implique :
(a) l'établissement d'un support à l'intérieur duquel le courant de stimulation le plus faible est contenu ; et
(b) coupe successivement le support en deux jusqu'à ce que le courant de stimulation le plus faible soit déterminé avec une précision spécifiée.

10. Système selon l'une quelconque des revendications 1 à 9, comprenant en outre un dispositif d'affichage en communication avec l'unité de commande (12) pour communiquer visuellement avec ledit utilisateur.

11. Système selon la revendication 10, dans lequel le dispositif d'affichage comprend des capacités de commande par écran tactile pour permettre à l'utilisateur d'interagir avec l'unité de commande (12).

12. Système selon l'une quelconque des revendications 1 à 11, dans lequel l'unité de commande (12) est en outre configurée pour effectuer au moins l'une des évaluations EMG et MEP stimulées.

13. Système selon l'une quelconque des revendications 1 à 12, dans lequel l'évaluation est communiquée par l'affichage d'au moins une cause possible d'un changement de relation entre la réponse somatosensorielle audit premier signal de stimulation et la réponse somatosensorielle ultérieure audit second signal de stimulation.
